# EUROPEAN PATENT SPECIFICATION

(11) **EP 3 886 847 B1**
(45) Date of publication and mention of the grant of the patent: **21.08.2024**
(21) Application number: 19808606.8
(22) Date of filing: 27.11.2019
(51) Int. Cl.: A61K 31/4245, A61K 31/4439, A61K 31/426, A61K 35/763, A61K 45/06, A61P 35/00, A61K 39/12, A61P 31/22, C12N 7/00

(54) **HELICASE PRIMASE INHIBITORS FOR TREATING CANCER IN A COMBINATION THERAPY WITH ONCOLYTIC VIRUSES**
HELIKASE-PRIMASE-INHIBITOREN ZUR BEHANDLUNG VON KREBS IN EINER KOMBINATIONSTHERAPIE MIT ONKOLYTISCHE VIREN
INHIBITEURS D'HÉLICASE-PRIMASE POUR LE TRAITEMENT DU CANCER DANS UNE THÉRAPIE COMBINÉE AVEC DES VIRUS ONCOLYTIQUES

(30) Priority: 28.11.2018 EP 18208756
(43) Date of publication of application: 06.10.2021
(73) Proprietor: Innovative Molecules GmbH, 32107 Bad Salzuflen (DE)
(72) Inventor: KLEYMANN, Gerald, 32107 Bad Salzuflen (DE); GEGE, Christian, 89584 Ehingen (DE)
(74) Representative: Gille Hrabal Partnerschaftsgesellschaft mbB Patentanwälte
(86) International application number: PCT/EP2019/082751
(87) International publication number: WO 2020/109389

(56) References cited:
- WO-A1-2017/174640
- D KOLODKIN-GAL ET AL: "Herpes simplex virus delivery to orthotopic rectal carcinoma results in an efficient and selective antitumor effect", GENE THERAPY, vol. 16, no. 7, 14 May 2009 (2009-05-14), GB, pages 905 - 915, XP055589479, ISSN: 0969-7128, DOI: 10.1038/gt.2009.44
- YAJIMA MISAKO ET AL: "Profile of anti-herpetic action of ASP2151 (amenamevir) as a helicase-primase inhibitor", ANTIVIRAL RESEARCH, ELSEVIER BV, NL, vol. 139, 24 December 2016 (2016-12-24), pages 95 - 101, XP029901946, ISSN: 0166-3542, DOI: 10.1016/J.ANTIVIRAL.2016.12.008
- QUENELLE DEBRA C ET AL: "Efficacy of pritelivir and acyclovir in the treatment of herpes simplex virus infections in a mouse model of herpes simplex encephalitis", ANTIVIRAL RESEARCH, vol. 149, 4 November 2017 (2017-11-04), pages 1 - 6, XP085315572, ISSN: 0166-3542, DOI: 10.1016/J.ANTIVIRAL.2017.11.002

## Description

### SUMMARY OF THE INVENTION

The present invention relates to the antiviral compounds as defined in the claims, which act as helicase primase inhibitors in a combination therapy with oncolytic herpesviruses for treating tumors, cancer or neoplasia.

### INTRODUCTION

The second most common cause of death in men and women is cancer. In men, prostate and lung cancer are most frequent, whereas women are most likely to develop breast cancer. The likelihood of developing cancer increases with age. The incidence is rare in youth, increases sharply from the age of 35 and reaches a prevalence of nearly 40% at the age of 75.

Cancer is a malignant tissue that can emanate from all cell types and affect all organs. The cause of cancer has not been finally clarified, but in most cases there is a change in the genetic material, which leads to an imbalance of cell growth and cell proliferation and thus to a change in natural cell death or growth control by surrounding cells or tissue. Normally these aberrant cells are destroyed by the immune system (elimination). The escape from the immune surveillance (immunosurveillance) and tolerance of tumors is called immunoediting and is defined as three subsequent steps or stages called elimination, equilibrium, and escape. A malignant tumor (neoplasia) occurs when abnormally growing cells (equilibrium) detach from a tissue structure and invade lymph and blood vessels and spread throughout the body (escape). The tumor is a mixture of different cell types, similar to an organ that communicates and interacts with the whole body. It forms a microenvironment of connective tissue, endothelial and muscle cells as well as cells of the immune system that enable it to survive, grow and spread throughout the body (metastasis). By an ingenious regulation of immune cells and formation of new blood vessels (angiogenesis), a tumor may go unnoticed by the immune system and spreads unhindered into surrounding layers of tissue (Owyong et al., Front. Cell Dev. Biol. 2018:6,19; Christiansen et al., PNAS 2011:108,4141).

Cancer is treated with numerous concepts and strategies such as surgery, radiotherapy, chemotherapy, immunotherapy, oncolytic viruses, immunovirotherapy etc. Sometimes the treatments and therapies listed are combined albeit in many cases the mono- or combination therapy is associated with significant side effects reducing the quality of life of the patients. Most therapies only extend life expectancy of cancer patients compared to untreated patients but recently cure in the range of 10-50% of the patients suffering from selected cancer types is reported in the field of checkpoint-inhibitors (a Nobel prize has been awarded in 2018 for discovery of the principle of immunotherapy with checkpoint inhibitors) and oncolytic viruses (oncolytic herpesviruses (oHSVes) have been approved in 2015 by the FDA). Oncolytic viruses are used to infect and kill the tumor cells and the immune system is controlling the viral infection and thereby also recognizing the tumor and subsequently destroying the primary infected tumor and also metastasis.

The main concern of the use of oncolytic viruses or pathogens in general is safety. Hence, laboratory strains, genetically modified or attenuated viruses or pathogens are used to avoid significant disease, chronic infection or latency caused by the pathogen; wild type viruses or clinical isolates thereby leading to morbidity or potentially death of the patient.

The pandemic of herpes infections has plagued humanity since ancient times, causing mucocutaneous infection such as herpes labialis and herpes genitalis. Disease symptoms often interfere with everyday activities and occasionally HSV infections are the cause of life-threatening (encephalitis) or sight-impairing disease (keratitis), especially in neonates, elderly and the immunocompromised patient population such as transplant or cancer patients or patients with an inherited immunodeficiency syndrome or disease. After infection the alpha herpesviridae persist for life in neurons of the host in a latent form, periodically reactivating and often resulting in significant psychosocial distress for the patient. Currently no cure is available.

So far, vaccines, interleukins, interferons, therapeutic proteins, antibodies, immunomodulators and small-molecule drugs with specific or non-specific modes of action lacked either efficacy or the required safety profile to replace the nucleosidic drugs acyclovir, valacyclovir and famciclovir as the first choice of treatment.

Due to safety concerns many oHSVes are modified by deletion of the thymidine kinase which is essential for establishing latency in neurons and reactivating from the latent stage. Unfortunately, this thymidine kinase (TK) activity is needed for activation of the nucleosides (such as the nucleosidic prodrugs acyclovir, valaciclovir, penciclovir and famciclovir etc.) to nucleotides in infected cells for treating the viral infection.

The known thiazolyl amides are the most potent anti-herpes drugs in development today. These antiviral agents act by a novel helicase primase mechanism of action and display low resistance rates *in vitro* and superior efficacy in animal models compared to nucleosidic drugs, however, development is hampered by off-target carbonic anhydrase activity and an unusual pharmacokinetic profile. The helicase primase inhibitor amenamevir is only launched for varicella zoster infections and displays low efficacy proabaly due to low brain exposure.

This patent application discloses the antiviral compounds as defined in the claims acting as helicase primase inhibitors for treating cancer in a cancer therapy using oncolytic herpesviruses. The preferred antiviral compounds of the present invention lack (or at least show significantly reduced) carbonic anhydrase activity, showing an improved solubility and a suitable pharmacokinetic profile for the new use of the present invention. Importantly the preferred helicase primase inhibitors of the present invention show exposure in the nervous system where the neurotrophic alpha-herpesviruses hide and reside and establish a chronic infection or latency.

The helicase primase inhibitors of the present invention, or in general the antidote presented in this application, allow for precise control of the oHSVes at any time post infection (pi) and even during repeated use or in immunocompromised patients where the use of oncolytic viruses is limited due to safety concerns.

Currently oHSVes are licensed for topical use only such as treatment for melanoma due to safety concerns. However, the real potential lies in the systemic treatment of solid tumors inside the body such as pancreas and/or colon carcinoma or glioblastoma and not only on the surface of the patient in case of melanoma.

In this case an antidote to terminate viral replication of oncolytic viruses such as oHSV used to treat cancer in any part of the body including the brain is a solution to said problem, namely, to precisely control the effect of oHSVes and finally end the viral replication when the desired treatment outcome on cancer has been accomplished.

### PRIOR ART

The prior art regarding the invention and use of oHSVes is reviewed by Bommareddy et al. (Annu. Rev. Cancer Biol. 2018:2,155). Animal models used to demonstrate efficacy of oHSV are reviewed by Speranza et al. (ILAR J. 2016:57,63). The use of oHSV in combination with nucleosides such as ganciclovir or acyclovir is described by Kolodkin-Gal et al. (J. Virol. 2008:82,999) or (Gene Ther. 2009:16,905). Acyclovir was used as a tool compound in cell or organ culture but not in animal models in the J. Virol. publication to prove diverse hypotheses. The Herpes thymidine kinase converts ganciclovir to a toxic chemotherapeutic such as conventional chemotherapy and besides this it's antiviral activity is associated with hematotoxic side effects. This HSV-TK suicide gene strategy converting ganciclovir to a chemotherapeutic agent has also been employed to other viruses such as adeno- or sindbis-viruses.

The first attenuated and genetically engineered oHSV (T-Vec/imlygic) has been launched for topical treatment of human melanoma in 2015/2016. Due to safety concerns the oHSVes are limited to topical treatment. Wild-type and attenuated and/or genetically engineered oHSVes are extensively studied in clinical trials.

oHSV have also been combined with chemotherapeutics (toxic or cytostatic drugs) to enhance the tumor cell killing properties of the individual components used by combination therapy. The oHSVes were also used in combination with nucleosides or nucleotides. However, some nucleotides were withdrawn from the market due to toxicity and nucleosides such as acyclovir, valacyclovir, penciclovir, famciclovir, brivudine are only moderately active at high doses and only marginal efficacious in treating herpes encephalitis due to insufficient or high IC₅₀ in oHSV infected cell culture and high ED₅₀ values in animal models demonstrating the need for more potent and efficacious antiviral therapy.

Wildtype or genetically engineered oncolytic HSV vectors expressing proteins with immune system modulating activity, oHSV infected cells, oHSV treatment of cancer, diverse applications of oHSV such as topical application, infusions or intratumoral injections and combinations of the oHSV with checkpoint inhibitors or chemotherapeutic agents to enhance the tumor killing activity of either the immunotherapy, chemotherapy or the oHSV infection have been described in diverse patent applications: AU2014342465, AU2007277703, AU2003258060, AU2002307795, AU2001026947, AU2001226951, AU1999029051, CA2928956, CA2846372, CA2634591, CA2479763, CA2398343, CA2398335, CN106068326, CN106551939, CN106974942, CN104877969, CN102206613, CN102051379, CN101768576, CN101671656, CN101560502, CN1865449, CN1425073, CN1418255, DK1252323, DK1252322, EP3371599, EP3324988, EP3268466, EP3217993, EP3184641, EP3082834, EP3063279, EP2849852, EP2753355, EP2662117, EP2307033, EP2281897, EP2177619, EP1979000, EP1685254, EP1568779, EP1494613, EP1487983, EP1406668, EP1252323, EP1252322, GB2537053, GB2516521, GB2501991, GB2374873, GB2375113, IN201617014813, JP2015221813, JP2015057054, KR1020030032913, KR1020020080383, MX2016005488, MXPA/a/2002/007061, MXPA/a/2002/007061, US2018/250352, US2018/207212, US2018/169241, US2018/071348, US2017/319638, US2017/274025, US2017/216381, US2017/035819, US2016/303174, US2016/250267, US2015/250837, US2015/232812, US2015/125425, US2015/110822, US2014/154216, US2014/154215, US2013/202639, US2013/034586, US2012/321599, US2012/164108, US2012/100109, US2011/236415, US2011/177032, US2009/311664, US2009/285860, US2009/220460, US2007/264282, US2007/003571, US2006/039894, US2004/120928, US2004/009604, US2003/113348, US2003/091537, US6428968, WO2018/115458, WO2018/118819, WO2018/026872, WO2017/218689, WO2017/189754, WO2017/181420, WO2017/143308, WO2017/076880, WO2017/013419, WO2017/013421, WO2016/146535, WO2015/089280, WO2015/066042, WO2014/047350, WO2013/190290, WO2013/167909, WO2013/036795, WO2012/056327, WO2011/119925, WO2009/148488, WO2008/013276, WO2008/008276, WO2007/075879, WO2007/052029, WO2006/002394, WO2005/049845, WO2003/082200, WO2003/073918, WO2002/087625, WO2001/053506, WO2001/053505 and WO2000/054795.

To date helicase primase inhibitors have not been applied in combination with oHSV to precisely control the replication of oHSV used for cancer therapy. Surprisingly, the helicase primase inhibitors described herein, such as in particular those of the Formulae (**I**), (**Ia**) and **(Ib)** turned out as a suitable antidote in the new use for treating cancer as described herein. The particularly used Example 7(-) further showed surprisingly new features such as the lowest ED₅₀ in animal models reported so far and due to its unique highest brain exposure reported to date can not only be used in combination with oHSV topically but also with oHSV injected systemically or intratumorally in any part of the body of the animal or human in need. Due to the high brain exposure even treatment of brain tumors such as the deadly glioblastoma may become treatable by precise control of the oHSV replication in the tumor with helicase primase inhibitors.

The preferred helicase primase inhibitors of the Formulae (**I**), (**Ia**) and **(Ib)** of the present invention have been described in WO2017/174640 and WO2019/068817. The most prominent example is the following structure:

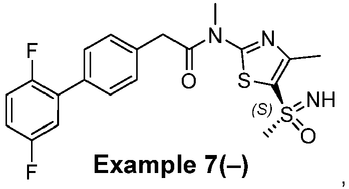

The *(S)*-configuration of the stereocenter was confirmed by X-ray analysis of a closed analog. Patent application WO2019/068817 describes beneficial properties of this helicase primase inhibitor in more detail, e.g. improved brain penetration and no off-target carboanhydrase activity which is known for primary sulfonamides.

Unexpectedly the helicase primase inhibitors of the Formulae (**I**), (**Ia**) and **(Ib)** show high brain exposure compared to other conventional antiviral drugs and in particular anti-herpes compounds used to date. In case of alpha-herpes therapy this is of most importance, since alpha-herpesviruses establish latency in the nervous system. In other words, though these viruses hide an reside in nerve cells of the infected host treatment becomes feasible even in this hidden reservoir of herpesviruses due to high concentrations of the drug at this most relevant site.

Nucleosidic drugs may be used - however, brain exposure is low and thus they show only low to moderate efficacy in an encephalitis model (at least one order of magnitude less potent than helicase primase inhibitors), high doses (gram amounts) have to be applied in patients and significant toxicity is observed in the case of treatment with ganciclovir.

The optimised pharmacokinetic profile of the helicase primase inhibitors, such as in particular those of the preferred compounds of the present invention, leads to a profound antiviral activity in treated mammals suitable for clinical development in humans and application as an antidote to control oncolytic herpesviruses used to treat cancer and are thus particularly suitable in a combination therapy with oncolytic herpesviruses for treating cancer.

So far, the use of helicase primase inhibitors, and in particular of those described in the present application, has not been described to control oncolytic viruses in general or oHSV in particular for safe and improved use in the treatment of cancer, especially for control of oHSV used for systemic oHSV therapy or intratumoral oHSV injections to treat cancer.

### DETAILED DESCRIPTION OF THE INVENTION

The present invention relates to the helicase primase inhibitors according to the claims in a combination therapy with oncolytic herpesviruses for treating cancer. Helicase primase inhibitors of the present invention are represented by the structure of the Formula **(I)** 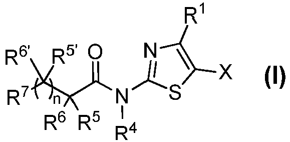
an enantiomer, diastereomer, tautomer, *N*-oxide, solvate, formulation or pharmaceutically acceptable salt thereof, wherein in Formula (I)
X is selected from 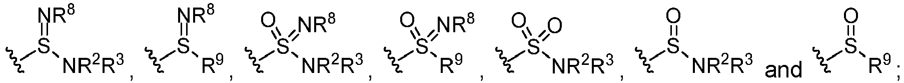
R¹ is selected from H, halogen, C₁₋₆-alkyl, halo-C₁₋₆-alkyl, hydroxy-C₁₋₆-alkyl, C₃₋₆-cycloalkyl, halo-C₃₋₆-cycloalkyl, -O-C₁₋₆-alkyl, -O-halo-C₁₋₆-alkyl and -NH-C₁₋₆-alkyl;
R² is selected from H, -CN, -NO₂, C₁₋₁₀-alkyl, C₂₋₁₀-alkenyl, C₂₋₁₀-alkynyl, C_{O-10}-alkylene-C₃₋₁₀-cycloalkyl, C₀₋₁₀-alkylene-C₃₋₁₀-heterocycloalkyl, C₀₋₁₀-alkylene-(5- to 10-membered heteroaryl), C₀₋₁₀-alkylene-(6- to 10-membered aryl), C₀₋₁₀-alkylene-(6- to 10-membered heteroaryl), C₀₋₁₀-alkylene-OR¹¹, C₀₋₁₀-alkylene-CO₂R¹¹, C₀₋₁₀-alkylene-C(=O)NR¹¹R¹², C₀₋₁₀-alkylene-C(=S)NR¹¹R¹², C₀₋₁₀-alkylene-C(=O)NR¹¹SO₂R¹³, C₀₋₁₀-alkylene-C(=S)NR¹¹SO₂R¹¹, C₀₋₁₀-alkylene-C(=O)R¹¹, C₀₋₁₀-alkylene-C(=S)R¹¹, C₀₋₁₀-alkylene-SR¹¹, C₀₋₁₀-alkylene-SOₓR¹³, C₀₋₁₀-alkylene-SO₃R¹¹, C₀₋₁₀-alkylene-SO₂NR¹¹R¹², C₀₋₁₀-alkylene-NR¹¹C(=O)R¹¹, C₀₋₁₀-alkylene-NR¹¹C(=S)R¹¹, C₀₋₁₀-alkylene-NR¹¹SO₂R¹³, C₀₋₁₀-alkylene-NR¹¹C(=O)NR¹¹R¹², C₀₋₁₀-alkylene-NR¹¹C(=S)NR¹¹R¹², C₀₋₁₀-alkylene-NR¹¹SO₂NR¹¹R¹², C₀₋₁₀-alkylene-NR¹¹R¹², wherein alkyl, alkenyl, alkynyl, alkylene, cycloalkyl, heterocycloalkyl, aryl and heteroaryl is unsubstituted or substituted with 1 to 7 substituents independently selected from the group consisting of oxo, CN, -NO₂, OR¹¹, O-C₂₋₆-alkylene-OR¹¹, C₁₋₆-alkyl, halo-C₁₋₆-alkyl, halogen, CO₂R¹¹, C(=O)NR¹¹R¹², C(=O)NR¹¹SO₂R¹¹, C(=O)R¹¹, SR¹¹, SOₓR¹¹, SO₃R¹¹, P(=O)(OR¹¹)₂, SO₂NR¹¹R¹², NR¹¹C(=O)R¹¹, NR¹¹SO₂R¹³, NR¹¹C(=O)NR¹¹R¹², NR¹¹SO₂NR¹¹R¹², C₃₋₁₀-cycloalkyl, O-C₃₋₁₀-cycloalkyl, C₃₋₁₀-heterocycloalkyl, O-C₃₋₁₀-heterocycloalkyl and NR¹¹R¹²;
R³ is selected from H, C₁₋₆-alkyl, halo-C₁₋₆-alkyl, -O-C₁₋₆-alkyl, -O-halo-C₁₋₆-alkyl, C₃₋₆-cycloalkyl and C₃₋₆-heterocycloalkyl, wherein alkyl, cycloalkyl and heterocycloalkyl are optionally substituted with 1 to 5 substituents independently selected from halogen, -CN, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
or R² and R³ when taken together with the nitrogen to which they are attached complete a 3- to 8-membered ring containing carbon atoms and optionally containing 1 or 2 heteroatoms selected from O, S or N, wherein the ring is unsubstituted or substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
R⁴ is selected from H, C₁₋₆-alkyl, C₁₋₆-acyl, C₂₋₆-alkenyl, C₃₋₈-cycloalkyl and C₃₋₈-heterocycloalkyl, wherein alkyl, acyl, alkenyl, cycloalkyl and heterocycloalkyl are optionally substituted with 1 to 5 substituents independently selected from halogen, -CN, OH, oxo, C₁₋₃-alkyl, halo-C₁₋₃-alkyl, O-C₁₋₃-alkyl and O-halo-C₁₋₃-alkyl;
R⁵ and R⁶ and R^{5'} and R^{6'} are independently selected from H, halogen, C₁₋₆-alkyl, NH₂, NHC₁₋₆-alkyl, N(C₁₋₆-alkyl)₂ and C₀₋₆-alkylene-C(=O)NH₂;
or R⁵ and R⁶ and R^{5'} and R^{6'} independently when taken together with the carbon to which they are attached complete a 3- to 8-membered ring containing carbon atoms and optionally containing 1 or 2 heteroatoms selected from O, S or N, wherein the ring is unsubstituted or substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
or R⁵ and R^{5'} and R⁶ and R^{6'} independently when taken together with the two adjacent carbon to which they are attached complete a 3- to 8-membered ring containing carbon atoms and optionally containing 1 or 2 heteroatoms selected from O, S or N, wherein the ring is unsubstituted or substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
or anyone of R⁵, R^{5'}, R⁶ and R^{6'} together with R⁷ may form an 8- to 11-membered bicyclic ring, which is optionally substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
R⁷ is selected from a 6-membered aryl and 5- or 6-membered heteroaryl, wherein aryl and heteroaryl are optionally substituted with 1 to 4 substituents independently selected from halogen, -CN, -NO₂, OH, C₁₋₆-alkyl, O-C₁₋₆-alkyl, C₃₋₆-cycloalkyl, O-C₃₋₆-cycloalkyl, C₃₋₆-heterocycloalkyl, O-C₃₋₆-heterocycloalkyl, SO_{y-}C₁₋₆-alkyl, CO₂H, C(=O)O-C₁₋₆-alkyl, 6- to 10-membered aryl, 5- to 10-membered heteroaryl, O-(6- to 10-membered aryl) and O-(5- to 10-membered heteroaryl), wherein alkyl, cycloalkyl, heterocycloalkyl, aryl and heteroaryl are optionally substituted with 1 to 5 substituents independently selected from halogen, -CN, - NO₂, OH, R¹³, OR¹³, CO₂R¹¹, NR¹¹R¹², C(=O)R¹¹, C(=S)R¹¹, C(=O)NR¹¹R¹², NR¹¹C(=O)NR¹¹R¹², NR¹¹C(=O)OR¹³, OC(=O)NR¹¹R¹², C(=S)NR¹¹R¹², NR¹¹C(=S)NR¹¹R¹², NR¹¹C(=S)OR¹³, OC(=S)NR¹¹R¹²; SO_{y}-C₁₋₆-alkyl, SO_{y}-halo-C₁₋₆-alkyl, SR¹¹, SOₓR¹³, SO₃R¹¹, SO₂NR¹¹R¹², NR¹¹SO₂R¹³ and NR¹¹SO₂NR¹¹R¹²;
R⁸ is selected from H, -CN, -NO₂, C₁₋₁₀-alkyl, C₂₋₁₀-alkenyl, C₂₋₁₀-alkynyl, C₀₋₁₀-alkylene-C₃₋₁₀-cycloalkyl, C₀₋₁₀-alkylene-C₃₋₁₀-heterocycloalkyl, C₀₋₁₀-alkylene-(5- to 10-membered heteroaryl), C₀₋₁₀-alkylene-(6- to 10-membered aryl), C₀₋₁₀-alkylene-(6- to 10-membered heteroaryl), C₀₋₁₀-alkylene-OR¹¹, C₀₋₁₀-alkylene-CO₂R¹¹, C₀₋₁₀-alkylene-C(=O)NR¹¹R¹², C₀₋₁₀-alkylene-C(=S)NR¹¹R¹², C₀₋₁₀-alkylene-C(=O)NR¹¹SO₂R¹³, C₀₋₁₀-alkylene-C(=S)NR¹¹SO₂R¹¹, C₀₋₁₀-alkylene-C(=O)R¹¹, C₀₋₁₀-alkylene-C(=S)R¹¹, C₀₋₁₀-alkylene-SR¹¹, C₀₋₁₀-alkylene-SOₓ-R¹³, C₀₋₁₀-alkylene-SO₃R¹¹, C₀₋₁₀-alkylene-SO₂NR¹¹R¹², C₀₋₁₀-alkylene-NR¹¹C(=O)R¹¹, C₀₋₁₀-alkylene-NR¹¹C(=S)R¹¹, C₀₋₁₀-alkylene-NR¹¹SO₂R¹¹, C₀₋₁₀-alkylene-NR¹¹C(=O)NR¹¹R¹², C₀₋₁₀-alkylene-NR¹¹C(=S)NR¹¹R¹², C₀₋₁₀-alkylene-NR¹¹-SO₂-NR¹¹R¹², C₀₋₁₀-alkylene-NR¹¹R¹², wherein alkyl, alkenyl, alkynyl, alkylene, cycloalkyl, heterocycloalkyl, aryl and heteroaryl is unsubstituted or substituted with 1 to 7 substituents independently selected from the group consisting of oxo, CN, -NO₂, OR¹¹, O-C₂₋₆-alkylene-OR¹¹, C₁₋₆-alkyl, halo-C₁₋₆-alkyl, halogen, CO₂R¹¹, CONR¹¹R¹², CONR¹¹SO₂R¹¹, COR¹¹, SOₓR¹¹, SO₃H, PO(OH)₂, SO₂NR¹¹R¹², NR¹¹COR¹¹, NR¹¹SO₂R¹¹, NR¹¹-CO-NR¹¹R¹², NR¹¹-SO₂-NR¹¹R¹², C₃₋₁₀-cycloalkyl, O-C₃₋₁₀-cycloalkyl, C₃₋₁₀-heterocycloalkyl, O-C₃₋₁₀-heterocycloalkyl and NR¹¹R¹²;
R⁹ is selected from C₁₋₁₀-alkyl, C₂₋₁₀-alkenyl, C₂₋₁₀-alkynyl, C₀₋₁₀-alkylene-C₃₋₁₀-cycloalkyl, C₀₋₁₀-alkylene-C₃₋₁₀-heterocycloalkyl, C₀₋₁₀-alkylene-(5- to 10-membered heteroaryl), C₀₋₁₀-alkylene-(6- to 10-membered aryl), C₀₋₁₀-alkylene-(6- to 10-membered heteroaryl), C₀₋₁₀-alkylene-OR¹¹, C₀₋₁₀-alkylene-CO₂R¹¹, C₀₋₁₀-alkylene-C(=O)NR¹¹R¹², C₀₋₁₀-alkylene-C(=S)NR¹¹R¹², C₀₋₁₀₋ alkylene-C(=O)NR¹¹SO₂R¹³, C₀₋₁₀-alkylene-C(=S)NR¹¹SO₂R¹¹, C₀₋₁₀-alkylene-C(=O)R¹¹, C₀₋₁₀-alkylene-C(=S)R¹¹, C₀₋₁₀-alkylene-SR¹¹, C₀₋₁₀-alkylene-SOₓR¹³, C₀₋₁₀-alkylene-SO₃R¹¹, C₀₋₁₀-alkylene-SO₂NR¹¹R¹², C₀₋₁₀-alkylene-NR¹¹C(=0)R¹¹, C₀₋₁₀-alkylene-NR¹¹C(=S)R¹¹, C₀₋₁₀-alkylene-NR¹¹SO₂R¹³, C₀₋₁₀-alkylene-NR¹¹C(=O)NR¹¹R¹², C₀₋₁₀-alkylene-NR¹¹C(=S)NR¹¹R¹², C₀₋₁₀-alkylene-NR¹¹SO₂NR¹¹R¹², C₀₋₁₀-alkylene-NR¹¹R¹², wherein alkyl, alkenyl, alkynyl, alkylene, cycloalkyl, heterocycloalkyl, aryl and heteroaryl is unsubstituted or substituted with 1 to 7 substituents independently selected from the group consisting of oxo, CN, -NO₂, OR¹¹, O-C₂₋₆-alkylene-OR¹¹, C₁₋₆-alkyl, halo-C₁₋₆-alkyl, halogen, CO₂R¹¹, C(=O)NR¹¹R¹², C(=O)NR¹¹SO₂R¹¹, C(=O)R¹¹, SR¹¹, SOₓR¹¹, SO₃R¹¹, P(=O)(OR¹¹)₂, SO₂NR¹¹R¹², NR¹¹C(=O)R¹¹, NR¹¹SO₂R¹³, NR¹¹C(=O)NR¹¹R¹², NR¹¹SO₂NR¹¹R¹², C₃₋₁₀-cycloalkyl, O-C₃₋₁₀-cycloalkyl, C₃₋₁₀-heterocycloalkyl, O-C₃₋₁₀-heterocycloalkyl and NR¹¹R¹²;
R¹¹ is independently selected from H, C₁₋₆-alkyl, C₀₋₆-alkylene-C₃₋₁₀-cycloalkyl and C₀₋₆-alkylene-C₃₋₁₀-heterocycloalkyl, wherein alkyl, alkylene, cycloalkyl and heterocycloalkyl is unsubstituted or substituted with 1 to 6 substituents independently selected from the group consisting of halogen, -CN, OH, oxo, C₁₋₃-alkyl, halo-C₁₋₃-alkyl, O-C₁₋₃-alkyl, O-halo-C₁₋₃-alkyl, NH₂, NH(C₁₋₃-alkyl), N(C₁₋₃-alkyl)₂, C₃₋₆-heterocycloalkyl, C₃₋₆-cycloalkyl, SO₂-NHC₁₋₃-alkyl, SO₂-N(C₁₋₃-alkyl)₂ and SO₂-C₁₋₃-alkyl, wherein cycloalkyl and heterocycloalkyl is unsubstituted or substituted with 1 to 3 substituents independently selected from the group consisting of F, OH, oxo, CH₃, CHF₂ and CF₃;
R¹² is independently selected from H, C₁₋₆-alkyl, halo-C₁₋₆-alkyl and C₃₋₆-cycloalkyl;
or R¹¹ and R¹² when taken together with the nitrogen to which they are attached complete a 3-to 8-membered ring containing carbon atoms and optionally containing 1 or 2 heteroatoms selected from O, S or N, wherein the ring is unsubstituted or substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
R¹³ is independently selected from C₁₋₆-alkyl, C₀₋₆-alkylene-C₃₋₁₀-cycloalkyl and C₀₋₆-alkylene-C₃₋₁₀-heterocycloalkyl, wherein alkyl, alkylene, cycloalkyl and heterocycloalkyl is unsubstituted or substituted with 1 to 6 substituents independently selected from the group consisting of halogen, -CN, OH, oxo, C₁₋₃-alkyl, halo-C₁₋₃-alkyl, O-C₁₋₃-alkyl, O-halo-C₁₋₃-alkyl, NH₂, NH(C₁₋₃-alkyl), N(C₁₋₃-alkyl)₂, C₃₋₆-heterocycloalkyl, C₃₋₆-cycloalkyl, SO₂-NHC₁₋₃-alkyl, SO₂-N(C₁₋₃-alkyl)₂ and SO₂-C₁₋₃-alkyl, wherein cycloalkyl and heterocycloalkyl is unsubstituted or substituted with 1 to 3 substituents independently selected from the group consisting of F, OH, oxo, CH₃, CHF₂ and CF₃;
n is selected from 0 and 1;
x is independently selected from 1 and 2;
y is independently selected from 0, 1 and 2;
and wherein optionally R¹ is connected to one residue selected from R², R³, R⁸, R⁹ or R¹¹ to form a 5- to 8-membered heterocycle, which is optionally substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
or the helicase primase inhibitors are represented by a structure of the following Formula 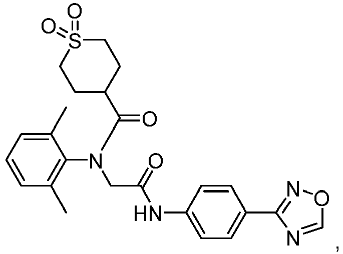
or a solvate, formulation or pharmaceutically acceptable salt thereof.

In the context of the present invention "C₁₋₁₀-alkyl" means a saturated alkyl chain having 1 to 10 carbon atoms which may be straight chained or branched. Examples thereof include methyl, ethyl, propyl, isopropyl, n-butyl, isobutyl, tert-butyl, n-pentyl, isopentyl, neopentyl, hexyl, heptyl, octyl, nonyl and decyl. Preferred is "C₁₋₆-alkyl", more preferred is "C₁₋₄-alkyl", most preferred is "C₁₋₃-alkyl".

The term "halo-C₁₋₁₀-alkyl" or "halo-C₁₋₆-alkyl", respectively, means that one or more hydrogen atoms in the alkyl chain are replaced by a halogen, as defined below. A preferred example thereof is the formation of a -CF₃ group.

The term "hydroxy-C₁₋₆-alkyl" means that one or more hydrogen atoms in the alkyl chain, as defined above, are replaced by a hydroxyl group (-OH). Examples thereof include hydroxymethyl, 1-hydroxyethyl, 2-hydroxyethyl, 1,2-dihydroxyethyl, 3-hydroxypropyl, 2-hydroxypropyl, 1-hydroxypropyl, 1-hydroxypropan-2-yl, 2-hydroxypropan-2-yl, 2,3-dihydroxypropyl, 1,3-dihydroxypropan-2-yl, 3-hydroxy-2-methyl-propyl, 2-hydroxy-2-methyl-propyl, 1-hydroxy-2-methyl-propyl etc. A preferred example thereof is hydroxymethyl (-CH₂OH).

"C₂₋₁₀-alkenyl" means an alkyl chain having 1 to 10 carbon atoms which may be straight chained or branched, containing at least one carbon to carbon double bond. Examples thereof include ethenyl, propenyl, decenyl, 2-methylenehexyl and (2*E*,4*E*)-hexa-2,4-dienyl. Preferred is "C₂₋₆-alkenyl".

"C₂₋₁₀-alkynyl" means an alkyl chain having 1 to 10 carbon atoms which may be straight chained or branched, containing at least one carbon to carbon triple bond. Examples thereof include ethynyl, propynyl and decynyl. Preferred is "C₂₋₆-alkynyl".

A "C₀₋₁₀-alkylene" means that the respective group is divalent and connects the attached residue with the remaining part of the molecule. Moreover, in the context of the present invention, "C₀-alkylene" is meant to be represent a bond. Preferred is "C₀₋₆-alkylene".

A C₃₋₁₀-cycloalkyl group or C₃₋₁₀-carbocycle means a saturated or partially unsaturated mono-, bi-, spiro- or multicyclic ring system comprising 3 to 10 carbon atoms. Examples include cyclopropyl, cyclobutyl, cyclopentyl, cyclohexyl, cyclohexenyl, bicyclo[2.2.2]octyl, bicyclo[2.2.1]heptyl, adamantyl and pentacyclo[4.2.0.0^{2,5}.0^{3,8}.0^{4,7}]octyl. Preferred is a C₃₋₆-cycloalkyl group. More preferred is a cyclopropyl group.

A C₃₋₁₀-heterocycloalkyl group means a saturated or partially unsaturated 3- to 10-membered carbon mono-, bi-, spiro- or multicyclic ring wherein 1, 2 or 3 carbon atoms are replaced by 1, 2 or 3 heteroatoms, respectively, wherein the heteroatoms are independently selected from N, O, S, SO and SO₂. Examples thereof include epoxidyl, oxetanyl, pyrrolidinyl, tetrahydrofuranyl, piperidinyl, piperazinyl tetrahydropyranyl, 1,4-dioxanyl, morpholinyl, 4-quinuclidinyl, 1,4-dihydropyridinyl and 3,6-dihydro-2/*7*-thiopyranyl. The C₃₋₁₀-heterocycloalkyl group can be connected via a carbon or nitrogen atom. Preferred is a C₃₋₆-heterocycloalkyl group.

A 5- to 10-membered mono- or bicyclic heteroaromatic ring system (within the application also referred to as heteroaryl) containing up to 5 heteroatoms means a monocyclic heteroaromatic ring such as pyrrolyl, imidazolyl, furanyl, thiophenyl, pyridinyl, pyrimidinyl, pyrazinyl, pyrazolyl, oxazolyl, isoxazolyl, triazolyl, oxadiazolyl and thiadiazolyl. Preferred are 5- to 6-membered monocyclic heteroaromatic rings. It further means a bicyclic ring system wherein the heteroatom(s) may be present in one or both rings including the bridgehead atoms. Examples thereof include quinolinyl, isoquinolinyl, quinoxalinyl, benzimidazolyl, benzisoxazolyl, benzodioxanyl, benzofuranyl, benzoxazolyl, indolyl, indolizinyl and pyrazolo[1,5-a]pyrimidinyl. The nitrogen or sulphur atom of the heteroaryl system may also be optionally oxidized to the corresponding *N*-oxide, *S*-oxide or *S*,*S*-dioxide. If not stated otherwise, the heteroaryl system can be connected via a carbon or nitrogen atom. Examples for *N*-linked heterocycles are

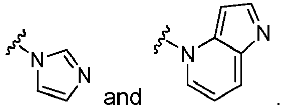

A 6- to 10-membered mono- or bicyclic aromatic ring system (within the application also referred to as aryl) means an aromatic carbon cycle such as phenyl or naphthalenyl. Preferred are 5- to 6-membered aromatic rings (aryl), such as in particular phenyl.

The term "5- to 8-membered heterocycle", which may be formed when R¹ is connected to one residue selected from R², R³, R⁸, R⁹ or R¹¹ contains the elements from Formula (I) and forms an optionally substitued partially saturated ring system, e.g.

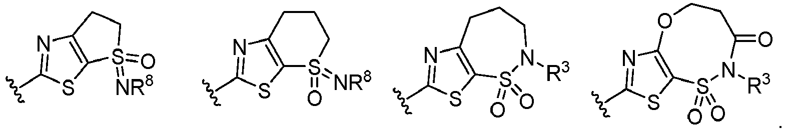

The term "*N*-oxide" denotes compounds, where the nitrogen in the heteroaromatic system (preferably pyridinyl) is oxidized. Such compounds can be obtained in a known manner by reacting a compound of the present invention (such as in a pyridinyl group) with H₂O₂ or a peracid in an inert solvent.

Halogen is selected from fluorine, chlorine, bromine and iodine, preferred are fluorine and chlorine.

Furthermore, the compounds of the present invention are partly subject to tautomerism. For example, if a heteroaromatic group containing a nitrogen atom in the ring is substituted with a hydroxy group on the carbon atom adjacent to the nitrogen atom, the following tautomerism can appear:

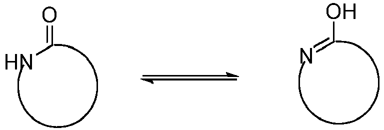

A C₃₋₁₀-cycloalkyl or C₃₋₁₀-heterocycloalkyl group can be connected straight or spirocyclic, e.g. when cyclohexane is substituted with the heterocycloalkyl group oxetane, the following structures are possible:

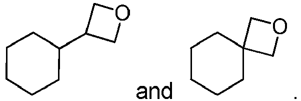

It will be appreciated by the skilled person that when lists of alternative substituents include members which, because of their valency requirements or other reasons, cannot be used to substitute a particular group, the list is intended to be read with the knowledge of the skilled person to include only those members of the list which are suitable for substituting the particular group.

The optical rotation (depicted as (-) or (+) in the text) used in the compound name and Example number relates to the measured value at 365 nm, if not stated otherwise.

The helicase primase inhibitor known under the name "Pritelivir" has the following structure and several polymorphs or salt forms are described in WO2013/045491, EP2573086, EP2602258, WO2013/045479, WO2018/095576, WO2018/096170 and WO2018/096177:

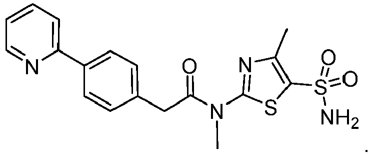

The mesylate monohydrate of pritelivir seems to be the most preferred salt.

The helicase primase inhibitor known under the name "Amenamevir" has the following structure and is described in WO2002/038554, WO2005/014559, WO2006/082821 and WO2009/123169:

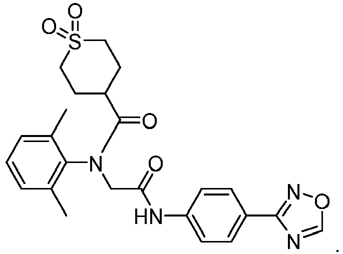

The compounds used or prepared in the present invention can be in the form of a pharmaceutically acceptable salt or a solvate. The term "pharmaceutically acceptable salts" refers to salts prepared from pharmaceutically acceptable non-toxic bases or acids, including inorganic bases or acids and organic bases or acids. In case the compounds of the present invention contain one or more acidic or basic groups, the invention also comprises their corresponding pharmaceutically or toxicologically acceptable salts, in particular their pharmaceutically utilizable salts. Thus, the compounds of the present invention which contain acidic groups can be used according to the invention, for example, as alkali metal salts, alkaline earth metal salts or ammonium salts. More precise examples of such salts include sodium salts, potassium salts, calcium salts, magnesium salts or salts with ammonia or organic amines such as, for example, ethylamine, ethanolamine, triethanolamine or amino acids. The compounds of the present invention which contain one or more basic groups, i.e. groups which can be protonated, can be used according to the invention in the form of their addition salts with inorganic or organic acids. Examples of suitable acids include hydrogen chloride, hydrogen bromide, phosphoric acid, sulfuric acid, nitric acid, methane sulfonic acid, p-toluene sulfonic acid, naphthalene disulfonic acids, oxalic acid, acetic acid, tartaric acid, lactic acid, salicylic acid, benzoic acid, formic acid, propionic acid, pivalic acid, diethyl acetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present invention simultaneously contain acidic and basic groups in the molecule, the invention also includes, in addition to the salt forms mentioned, inner salts or betaines (zwitterions). The respective salts can be obtained by customary methods which are known to the person skilled in the art like, for example, by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts. Depending on the substitution pattern, the specific compounds according to the invention can exist in stereoisomeric forms which either behave as image and mirror image (enantiomers), or which do not behave as image and mirror image (diastereomers). The invention relates both to the enantiomers or diastereomers and their respective mixtures. Like the diastereomers, the racemic forms can be separated into the stereoisomerically uniform components in a known manner.

In one aspect not covered by the wording of the claims, the present disclosure includes those compounds which are only converted into the actual active compounds of the Formulae (**I**), (**Ia**) and **(Ib)**, once inside the body (so-called prodrugs). Preferred embodiments of the present invention relate to the following helicase primase inhibitors:
In a particularly preferred embodiment of the invention the helicase primase inhibitors are represented by the Formula (**Ia**) and/or **(Ib)**: 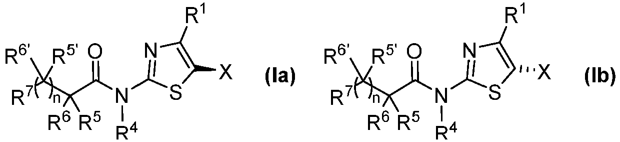
wherein the substituents have the meaning as defined *supra,*
or a solvate, formulation or pharmaceutically acceptable salt thereof.

In a further preferred embodiment in combination with any of the above or below embodiments the helicase primase inhibitors are represented by the Formula (**Ia**) and/or **(Ib),** 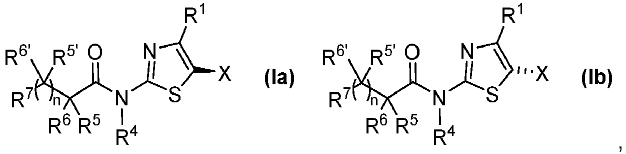 wherein
in the Formula (**Ia**) X is 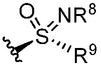 and in the Formula **(Ib)** X is 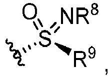 or a solvate, formulation or pharmaceutically acceptable salt thereof.

In a further more preferred embodiment in combination with any of the above or below embodiments n is 0.

In a further more preferred embodiment in combination with any of the above or below embodiments the helicase primase inhibitors are represented by the Formula 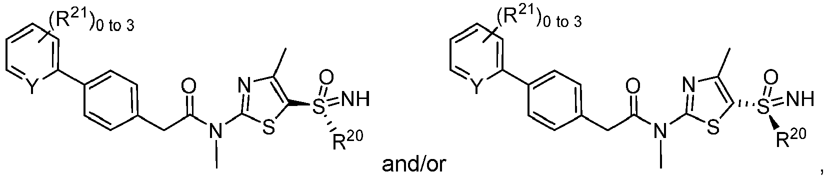 wherein
R²⁰ is selected from C₁₋₄-alkyl and C₃₋₆-cycloalkyl, wherein alkyl and cycloalkyl is unsubstituted or substituted with 1 to 3 substituents independently selected from the group consisting of F or Me;
R²¹ is selected from F, Cl, OH, Me, OMe, CHF₂, CF₃, OCHF₂, OCF₃; and
Y is selected from nitrogen or carbon,
or a solvate, formulation or pharmaceutically acceptable salt thereof.

In a further more preferred embodiment in combination with any of the above or below embodiments the helicase primase inhibitor is selected from the group consisting of 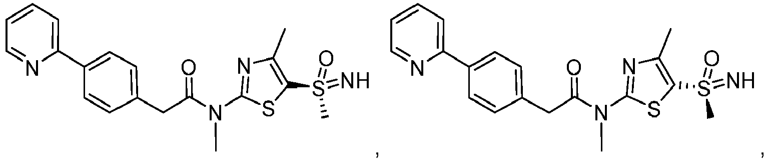 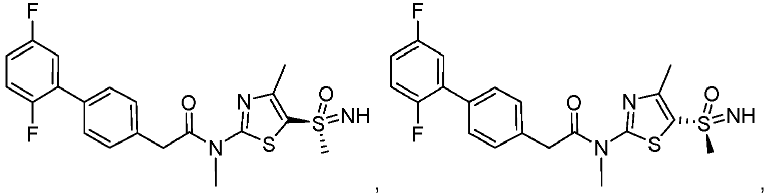 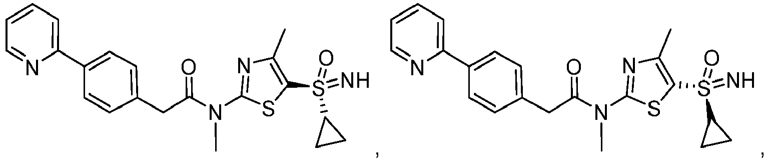 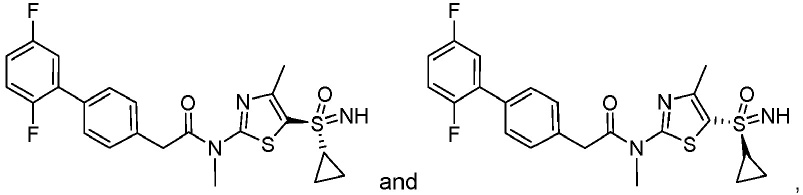 or a solvate, formulation or pharmaceutically acceptable salt thereof.

In a further more preferred embodiment in combination with any of the above or below embodiments the helicase primase inhibitor is selected from the group consisting of
(-)-*N*-Methyl-*N*-(4-methyl-5-(S-methylsulfonimidoyl)thiazol-2-yl)-2-(4-(pyridin-2-yl)phenyl)acetamide,
(-)-(*S*)-2-(2',5'-Difluoro-[1,1'-biphenyl]-4-yl)-*N*-methyl-*N*-(4-methyl-5-(*S-*methylsulfonimidoyl)thiazol-2-yl)acetamide,
(-)-*N*-(5-(Cyclopropanesulfonimidoyl)-4-methylthiazol-2-yl)-*N*-methyl-2-(4-(pyridin-2-yl)phenyl)acetamide, and
(-)-*N*-(5-(Cyclopropanesulfonimidoyl)-4-methylthiazol-2-yl)-2-(2',5'-difluoro-[1,1'-biphenyl]-4-yl)-*N*-methylacetamide.

In an alternatively preferred embodiment in combination with any of the above or below embodiments the helicase primase inhibitor is selected from 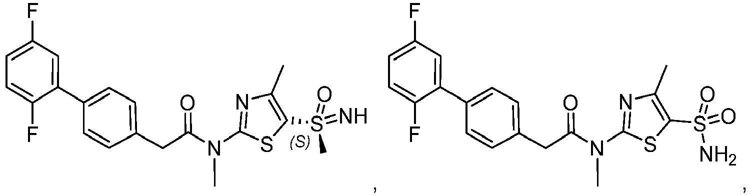 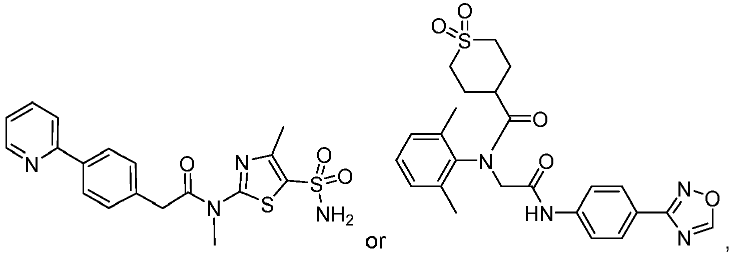 or a solvate, formulation or pharmaceutically acceptable salt thereof.

In a more preferred embodiment in combination with any of the above or below embodiments the helicase primase inhibitor is 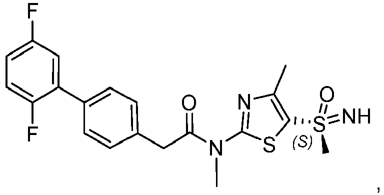 or a solvate, formulation or pharmaceutically acceptable salt thereof.

In an alternatively more preferred embodiment in combination with any of the above or below embodiments the helicase primase inhibitor is 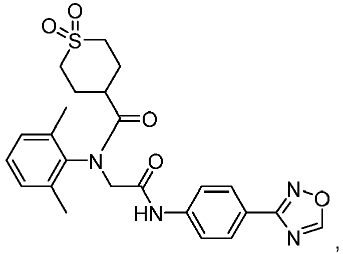 or a solvate, formulation or pharmaceutically acceptable salt thereof.

In an alternatively preferred embodiment in combination with any of the above or below embodiments the helicase primase inhibitor is amenamevir.

In yet another alternatively more preferred embodiment in combination with any of the above or below embodiments the helicase primase inhibitor is 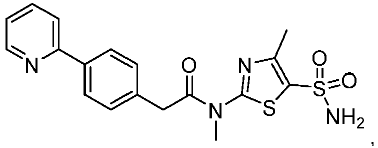 or a solvate, formulation or pharmaceutically acceptable salt thereof.

In yet another alternatively preferred embodiment in combination with any of the above or below embodiments the helicase primase inhibitor is pritelivir mesylate monohydrate.

The present invention relates to the described helicase primase inhibitors for use in the treatment and prophylaxis of cancer therapy by oncolytic herpesviruses in combination with said compounds to precisely control the activity of the oncolytic herpesviruses.

In a further aspect the invention relates to the described compounds of the present invention for use as an antidote for the oncolytic herpesviruses used in the treatment and prophylaxis of cancer by controlling the activity of oncolytic herpesviruses and/or by controlling, treating or preventing viral infections caused by the used oncolytic herpesviruses as an undesired side-effect of the cancer treatment.

In the sense of the present invention the term "antidote" refers to a compound having activity do control and in particular diminish and/or inactivate the oncolytic herpes virus used in the treatment or prophylaxis of cancer.

A further aspect of the present invention relates to a pharmaceutical composition for the use in a combination therapy with oncolytic herpesviruses for the treatment or prophylaxis of cancer according to the present invention, wherein said pharmaceutical composition comprises at least one helicase primase inhibitor according to the formula (I) as defined anywhere herein.

Said pharmaceutical composition may be used as an antidote in the combination therapy of the present invention, wherein said pharmaceutical composition and/or the helicase primase inhibitor comprised therein, acts to control, modulate, inhibit or shut off the activity of the oncolytic herpesviruses used in cancer therapy and which are sensitive to said inhibitors.

The pharmaceutical compositions described *supra* may further comprise at least one pharmaceutically acceptable carrier and/or at least one excipient and/or at least one further active substance, such as in particular antiviral active or immune modulating compounds, including checkpoint inhibitors, being effective in treating a disease or disorder associated with oncolytic herpesviruses infections used in the treatment of cancer.

The cancer to be treated in the combination therapy according to the present invention is preferably solid cancer.

The cancer to be treated in the combination therapy according to the present invention may be a cancer disease selected from liver cancer, lung cancer, colon cancer, pancreas cancer, kidney cancer, brain cancer, melanoma and glioblastoma etc.

The oncolytic viruses used in the combination therapy according to the present invention are oncolytic herpesviruses.

The oncolytic herpesviruses used in the combination therapy according to the present invention comprise oncolytic herpesviruses and oncolytic herpesviruses infected cells, which are preferably selected from an oncolytic wildtype, a clinical isolate or a laboratory herpesvirus strain or a genetically engineered or multi-mutated optionally attenuated or boosted oncolytic herpesvirus.

A further aspect of the present invention relates to a kit (kit-of-parts or combination preparation) comprising at least one of the helicase primase inhibitors according to formula (I) or the pharmaceutical composition comprising the same as described anywhere herein and at least one oncolytic herpesvirus. Such oncolytic herpesvirus is preferably one selected from a wildtype, a laboratory strain, a clinical isolate and a genetically engineered or multi-mutated oncolytic herpesvirus. In said kit the helicase primase inhibitor(s) or the pharmaceutical composition comprising the same are arranged (packed) separated from the at least one oncolytic herpesvirus. Suitable kit-of-parts arrangements are in principle well-known.

In accordance with the present invention such kit is for use in the treatment or prophylaxis of cancer as described anywhere herein.

A further aspect of the present invention relates to the pharmaceutical composition and/or of the helicase primase inhibitors according to formula (I) described *supra* for the use in a combination therapy with oncolytic herpesviruses for the treatment and prophylaxis of oncolytic herpes infections in cancer patients potentially with a suppressed immune system, such as AIDS patients, patients having a genetic or inherited immunodeficiency, transplant patients; in new-born children and infants; in herpes-positive patients, in particular oncolytic herpes-simplex-positive patients, for suppressing recurrence or oncolytic viral shedding; patients, in particular in herpes-positive patients, in particular oncolytic herpes-simplex-positive patients, who are resistant to nucleosidic antiviral therapy such as acyclovir, penciclovir, famciclovir, ganciclovir, valacyclovir and/or foscarnet or cidofovir. Such oncolytic herpes infections may occur as an undesired side effect in the cancer treatment with the oncolytic herpesviruses.

The helicase primase inhibitors or the pharmaceutical composition as described anywhere herein can be administered in the therapeutic use of the present invention by infusion, injection, intratumoral injection or topical or transdermal application of the oncolytic herpesviruses or oncolytic herpesvirus infected cells and/or of the helicase primase inhibitors or the pharmaceutical composition comprising the same.

According to the present invention a compound being active as a helicase primase inhibitor is characterized by an IC₅₀ value (HSV-1/Vero) in an *in vitro* activity selectivity assay HSV-1 on Vero cells as described in the Examples of the present invention of preferably IC₅₀ below 100 µM, more preferably IC₅₀ below 10 µM and very particularly preferable IC₅₀ below 1 µM.

According to the present invention a compound being active as a helicase primase inhibitor is characterized by an ED₅₀ value in an *in vivo* animal model as described in the Examples of the present invention preferably of ED₅₀ of less than 10 mg/kg for HSV-1 or HSV-2, more preferably of less than 5 mg/kg for HSV-1, and very particularly preferable of less than 2 mg/kg for HSV-1.

The preferred helicase primase inhibitors of the present invention are preferably characterized by showing no or reduced carbonic anhydrase inhibition, such particularly inhibition of carbonic anhydrase I and/or carbonic anhydrase II. In the sense of the present invention no or reduced carbonic anhydrase inhibition is particularly defined by IC₅₀-values (inhibitory concentration) in a carbonic anhydrase II activity assay according to R. Iyer et al. J. Biomol. Screen. 2006:11,782 and/or in a carbonic anhydrase I activity assay according to A. R. Katritzky et al. J. Med. Chem. 1987:30,2058 of IC₅₀ >2.0 µM, preferably >3.0 µM, more preferably >5.0 µM. Even more preferably, no or reduced carbonic anhydrase inhibition in the sense of the present invention is particularly defined by IC₅₀-values (inhibitory concentration) in a human carbonic anhydrase II activity assay of IC₅₀ >2.0 µM, preferably >3.0 µM, more preferably >5.0 µM and most preferably >10 µM.

The helicace primase inhibitors or pharmaceutical compositions of the present invention are considered for the use in the prophylaxis and treatment of cancer in humans as well as in animals.

The oncolytic herpesviruses used in the combination therapy of the present invention are herpesviruses encoding a helicase and/or primase by inhibiting the helicase and/or primase enzymes.

More specifically the oncolytic herpesviruses used in the combination therapy of the present invention are herpesviruses which nucleic acid encodes a helicase and/or primase and the related enzymes can be inhibited by the helicace primase inhibitors of the present invention, preferably at concentrations below 100 µM in vitro.

Specific examples for oncolytic herpesviruses comprise herpes simplex viruses or more particular HHV1 also named HSV-1 and/or HHV2 also named HSV-2. Particular examples of the oncolytic herpesviruses are the wildtype stain such as HSV-1 KOS strain, a laboratory strain such as HSV-1 strain (F) or a clinical isolate obtained from patient herpetic vesicles such as HSV-1 strain 17, attenuated multi-mutated herpes simplex virus such as HSV-1 G207 and a genetically engineered oncolytic virus such as talimogen laherparepvec (T-Vec).

Further, in a non-claimed aspect, the invention relates to a method of treating cancer with oncolytic herpesviruses in combination with the helicase primase inhibitor according to formula (I) as described anywhere herein or a disorder which is associated with oncolytic herpesviral infections (as a side effect of the cancer treatment) such as herpes disease, said methods comprising administering to a human or animal in need thereof an effective amount of a helicase primase inhibitor of the present invention or of a pharmaceutical composition comprising at least one of said helicase primase inhibitor as described anywhere herein.

In practical use, the helicace primase inhibitors used in the present invention can be combined as the active ingredient in intimate admixture with a pharmaceutical carrier according to conventional pharmaceutical compounding techniques. The carrier may take a wide variety of forms depending on the form of preparation desired for administration, e.g., oral, topical or parenteral (including intravenous). In preparing the compositions for oral dosage form, any of the usual pharmaceutical media may be employed, such as, for example, water, glycols, oils, alcohols, flavouring agents, preservatives, colouring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or non-aqueous techniques. Such compositions and preparations should contain at least 0.1 percent of the helicace primase inhibitor(s). The percentage of active helicace primase inhibitor in these compositions may, of course, be varied and may conveniently be between about 2 percent to about 60 percent of the weight of the unit. The amount of the helicace primase inhibitor(s) in such therapeutically useful compositions is such that an effective dosage will be obtained. The the helicace primase inhibitor(s) can also be administered intranasally as, for example, liquid drops or spray or as eye drops.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavouring such as cherry or orange flavour.

The helicace primase inhibitor(s) used in the present invention may also be administered parenterally. Solutions or suspensions of the helicace primase inhibitor(s) can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Any suitable route of administration may be employed for providing a mammal, especially a human, with an effective dose of the helicace primase inhibitor(s) of the present invention. For example, oral, rectal, topical, parenteral (including intravenous), ocular, pulmonary, nasal, and the like may be employed. Dosage forms include tablets, troches, dispersions, suspensions, solutions, capsules, creams, ointments, aerosols, and the like. Preferably the helicace primase inhibitor(s) of the present invention are administered orally or as eye drops, more preferably the compounds of the present invention are administered orally.

The effective dosage of the helicace primase inhibitor(s) employed may vary depending on the particular helicace primase inhibitor employed, the mode of administration, the condition being treated and the severity of the condition being treated. Such dosage may be ascertained readily by a person skilled in the art.

The helicace primase inhibitor(s) of the present invention may also be present in combination with further active ingredients, in particular with one or more active ingredients exhibiting advantageous effects in the treatment of any of the disorders or diseases as described herein. Very particularly the compounds of the present invention are present in a composition in combination with at least one further active substance being effective in treating a disease or disorder associated with viral infections (antiviral active compounds), preferably a disease or disorder being associated with viral infections caused by herpesviruses, such as in particular by herpes simplex viruses and/or with at least one further active substance being administered in the treatment or alleviation of the cancer disease and/or the treatment or alleviation of side-effects occurring in the cancer therapy. The at least one further active substance being effective in treating a disease or disorder associated with viral infections (antiviral active compounds) are preferably selected from the group consisting of nucleosidic drugs such as acyclovir, valacyclovir, penciclovir, ganciclovir, famciclovir and trifluridine, as well as compounds such as foscarnet and cidofovir or its ester cidofovir [(S)-HPMPC] bearing a hexaethyleneglycol moiety. Accordingly, the present invention further relates to a pharmaceutical composition comprising one or more of the helicace primase inhibitors of the present invention as described herein and at least one pharmaceutically acceptable carrier and/or excipient and/or at least one further active substance being effective in treating a disease or disorder associated with viral infections (antiviral active compounds) and/or being effective in the treatment or alleviation of the cancer disease and/or for treating or alleviating side-effects occurring in the cancer therapy.

### EXPERIMENTAL PART

The helicase primase inhibitors of the present invention, such as in particular the Example compound **7(-),** can be prepared by a combination of methods known in the artand/or by using the procedures described in WO2017/174640 and in WO2019/068817. The procedures can be applied to structures described in WO2018/127207:

### Example 1: 5-(2,5-Difluorophenyl)-N-methyl-N-(4-methyl-5-sulfamoylthiazol-2-yl)-2,3-di-hydro-1H-indene-2-carboxamide (1)

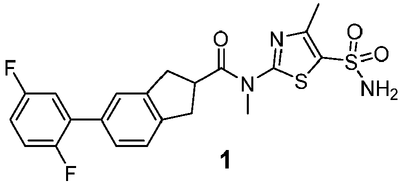

Starting from commercially available methyl 5-bromo-2,3-dihydro-1*H*-indene-2-carboxylate racemic target compound **1** was prepared similar as described in Example 21 in WO2018/127207 as a white solid. ¹H-NMR (400 MHz, DMSO-_{d6}) δ: 7.64 (s, 2H), 7.43 (s, 1H), 7.40-7.32 (m, 4H), 7.27-7.20 (m, 1H), 4.18-4.04 (m, 1H), 3.76 (s, 3H), 3.45-3.24 (m, 4H), 2.49 (s, 3H). MS found: 464.0 [M+H]⁺.

### Example 2 and 3: (-)-5-(2,5-Difluorophenyl)-N-methyl-N-(4-methyl-5-sulfamoylthiazol-2-yl)-2,3-dihydro-1H-indene-2-carboxamide (2) and (+)-5-(2,5-difluorophenyl)-N-methyl-N-(4-methyl-5-sulfamoylthiazol-2-yl)-2,3-dihydro-1H-indene-2-carboxamide (3)

The title compounds were prepared and further characterized by separation of the racemic mixture **1** by chiral SFC chromatography using the following conditions:
Stationary phase: OD-H (4.6*100 × 5 µm); injection volume: 5; co-solvent: IPA; column temperature 40°C; CO₂ flow rate: 2.4; co-solvent flow rate: 1.6; co-solvent: 40%; total flow: 4; front pressure: 163; back pressure 120; PDA start/stop wavelength 214/359 nm.

Example **2** is the first eluting enantiomer (retention time: 2.65 min). Said enantiomer is further characterized by a positive specific optical rotation of [α]²⁵_{589 nm} +110.81° (c = 0.879 g/100 mL, CH₃CN).

Example **3** is the second eluting enantiomer (retention time: 3.33 min). Said enantiomer is further characterized by a negative specific optical rotation of [α]²⁵_{589 nm} -101.61° (c = 1.018 g/100 mL, CH₃CN).

### Example 4:

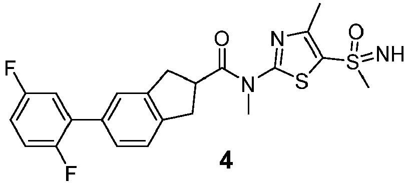

### Step 1: 5-(2,5-Difluorophenyl)-N-methyl-N-(4-methyl-5-(methylthio)thiazol-2-yl)-2,3-dihydro-1H-indene-2-carboxamide (4a)

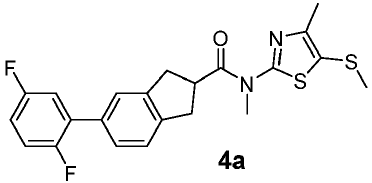

To a solution of 5-(2,5-difluorophenyl)-2,3-dihydro-1*H*-indene-2-carboxylic acid (550 mg, 2.00 mmol), HATU (1.14 g , 3.00 mmol) and Et₃N (606 mg, 6.00 mmol) in DMF (10 mL) was added *N*,4-dimethyl-5-(methylthio)thiazol-2-amine (348 mg, 2.00 mmol) at rt. The mixture was stirred overnight, diluted with in EA (30 mL) and washed with water (30 mL) twice. The organic layer was dried over Na₂SO₄, concentrated and purified by column chromatography on silica gel (CH₂Cl₂:EtOAc = 10:1) to give compound **4a** as a white solid.

### Step 2: 5-(2,5-Difluorophenyl)-N-methyl-N-(4-methyl-5-(methylsulfinyl)thiazol-2-yl)-2,3-dihydro-1H-indene-2-carboxamide (4b)

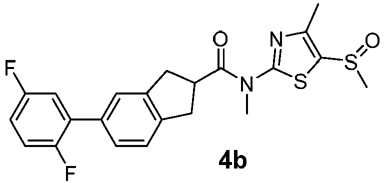

To a solution of compound **4a** (800 mg, 1.86 mmol) in CH₂Cl₂ (15 mL) was added *m*-CPBA (321 mg, purity 85%). The mixture was stirred at rt for 20 min and partitioned between CH₂Cl₂ and 5% sodium carbonate solution. The organic phase was washed with brine, dried over Na₂SO₄, concentrated and purified by column chromatography on silica gel (CH₂Cl₂:EtOAc = 1:1.5) to give compound **4b** as a white solid.

### Step 3: tert-Butyl ((2-(5-(2,5-difluorophenyl)-N-methyl-2,3-dihydro-1H-indene-2-carboxamido)-4-methylthiazol-5-yl)(methyl)(oxo)-I6-sulfaneylidene)carbamate (4c)

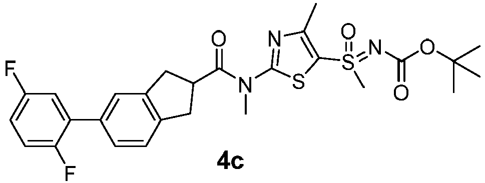

Magnesium oxide (269 mg, 6.72 mmol), *tert-*butyl carbamate (393 mg, 3.36 mmol), Rh₂(OAc)₄ (75 mg, 0.17 mmol) and (diacetoxy)iodobenzene (811 mg, 2.52 mmol) were added to a solution of compound **4b** (750 mg, 1.68 mmol) in CH₂Cl₂ (15 mL). The mixture was stirred at 40°C overnight, cooled to rt and filtered through a pad of Celite. The solvent was evaporated and the crude product was purified by column chromatography on silica gel (petroleum ether:EtOAc = 2:1) to give compound **4c** as a white solid.

### Step 4: 5-(2,5-Difluorophenyl)-N-methyl-N-(4-methyl-5-(S-methylsulfonimidoyl)thiazol-2-yl)-2,3-dihydro-1H-indene-2-carboxamide (4)

At ambient temperature, compound **4c** (800 mg, 1.43 mmol) was added to a stirred solution of trifluoroacetic acid (5 mL) in CH₂Cl₂ (10 mL) and stirring was continued for 3 h. The mixture was concentrated, then resolved in CH₂Cl₂, washed with saturated NaHCO₃ (2 × 40 mL), dried over Na₂SO₄, concentrated and purified by prep-TLC (petroleum ether:EtOAc = 1:1.5) to give compound **4** as a white solid. ¹H-NMR (400 MHz, DMSO-_{d6}) δ: 7.64 (s, 1H), 7.40-7.32 (m, 4H), 7.27-7.20 (m, 1H), 4.67 (br s, 1H), 4.14-4.06 (m, 1H), 3.76 (s, 3H), 3.45-3.37 (m, 2H), 3.30-3.24 (m, 2H), 3.14 (s, 3H), 2.53 (s, 3H). MS found: 462.1 [M+H]⁺.

### Example 5 to Example 9: 5-(2,5-Difluorophenyl)-N-methyl-N-(4-methyl-5-(S-methylsulfon-imidoyl)thiazol-2-yl)-2,3-dihydro-1H-indene-2-carboxamide (four possible stereoisomers)

The title compounds were prepared and further characterized by separation of the racemic mixture **4** by chiral SFC chromatography using the following conditions:
Stationary phase: AY-H (4.6*100 × 5 µm); injection volume: 5; co-solvent: EtOH/CH₃CN; column temperature 39.9°C; CO₂ flow rate: 2.2; co-solvent flow rate: 1.8; co-solvent: 45%; total flow: 4; front pressure: 155; back pressure 117; PDA start/stop wavelength 214/359 nm.
Example **5** is the first eluting enantiomer (retention time: 1.42 min);
Example **6** is the second eluting enantiomer (retention time: 1.97 min);
Example **8** is the third eluting enantiomer (retention time: 4.23 min);
Example **9** is the fourth eluting enantiomer (retention time: 7.77 min).

### Example 10: 2-(2',5'-Difluoro-[1,1'-biphenyl]-4-yl)-N-methyl-N-(4-methyl-5-sulfamoylthiazol-2-yl)acetamide (10)

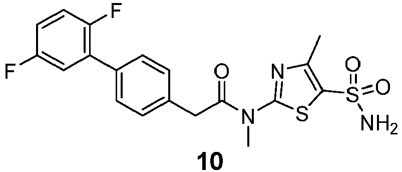

Compound **10** can be prepared as described in F. Carta et al. in J. Med. Chem. 2017:60,3154.

### In vitro Activity

The *in vitro* activity of the new compounds was measured as described in WO2017/174640 and in WO2019/068817.

| **Example** | IC₅₀ (HSV-1 infected Vero) | IC₅₀ (HSV-2 infected Vero) | IC₅₀ (HSV-1 ACV resistant) |
|---|---|---|---|
| Acyclovir | 0.5-3 µM | 0.5-3 µM | >25 µM |
| **7(-)** | 10-50 nM | 10-50 nM | 10-50 nM |
| **1** | 16 nM | 50 nM | 16 nM |
| **2** | 2 µM | 16 µM | 2 µM |
| **3** | 0.5 nM | 16 nM | 0.5 nM |
| **4** | 750 nM | 1.5 µM | 750 nM |
| **5** | 20 µM | 20 µM | 20 µM |
| **6** | 75 nM | 750 nM | 75 nM |
| **8** | 175 nM | 1.5 µM | 175 nM |
| **9** | 10 µM | 45 µM | 10 µM |

### In vivo Activity

### oHSV animal model

Assessment of the efficacy of an oncolytic virus treatment in the presence and absence of a potential virostatic test compound (Example **7(-))** in a syngeneic bilaterally-inoculated subcutaneous CT26 tumor-bearing mouse model.

### Experimental Design:

CT26 tumor cells (500 000 cell in 100 µL) were subcutaneously inoculated bilaterally into the right and left flanks of 9 week-old immuno-competent female Balbc/j mice after 20 days acclimation (Provider: Charles River Laboratories - BP 0109 - F 69592 L'Arbresle Cedex).

Ventilation and air treatment were performed through frequent turnover (25 volumes/hour depending on the density of animals housed) and temperature controlled around 21-22°C. Humidity was maintained around 50%. Artificial lighting was maintained 12 hours a day. Quantity and access to food (pellets) and drink (tap water) were checked daily.

Mice were housed in collective cages, with 4 animals per cage (530 cm² cage). Cages were renewed once a week by animal care taker.

Colorectal CT26 tumor cell line. Cells were cultured *in vitro* according to provider's specifications, i.e. RPMI 1640 supplemented with FBS at the final concentration of 10%.

Before inoculation in mice, cell viability was assessed by flow cytometry analysis and viable cell gating. A cell suspension was prepared according to the viable cell count.

The inoculation procedure of colorectal CT26 tumors in mice was as follows:
- At the end of the 20 days acclimation period, and in order to identify the animal all along the experimental procedure (for tumor volume monitoring in particular), mice were identified using tattoo.
- The day before tumor cell inoculation, mice have been shaved at the injection sites, under isoflurane gas anesthesia (4% induction and 2% maintain).
- The day of inoculation, mice were anesthetized with gas anesthesia with 4% isoflurane induction and 2% isoflurane for anesthesia maintaining.
- Tumor cells to be inoculated were resuspended in sterile PBS and the needed volume was loaded in 1 mL syringe with a 26 gauge needle (500000 cells/100 µL).

Virus injection (with HSV-1 Herpes Simplex Virus or vehicle) was performed with a 29G gauge needle (0.5 mL) insulin syringe when tumors reached an average volume of 40 mm³ (on day 7). At this time, virus was injected intratumorally (50 µL corresponding to 5×10⁶ HSV-1 (experimental groups 3, 5, 6 and 8) or 100 µL corresponding to 1×10⁸ HSV-1 (experimental groups 4 and 7)), into the tumor of the right flank.

The HSV-1 virus was provided as a stock concentration (in 1×10⁹ pfu/mL) vial. Virus vehicle either for virus stock solution dilution or for virus vehicle administration was DMEM medium 4.5 g/L glucose with glutamine (Lonza) + FBS 10% (Sigma Aldrich) + penicillin/streptomycin 1% (100 U) (Lonza) + HEPES 1 mM (Lonza) as indicated. In experimental group 2, 50 µL of virus vehicle was injected into the tumor of the right flank.

Pharmacological animal groups (8 animal per group, 64 animals in total) were organized as such:
1. **Control virus-untreated group:** test compound vehicle (200 µL, 5% DMSO; 0.5% hydroxypropylmethylcellulose (HPMC) in PBS) oral gavage (20 gauge needle standard suspension applicator), once a day, for 10 days and starting 72 h after virus injection for the other groups.
2. **Control virus vehicle-treated group: 50 µL of virus vehicle,** intratumoral injection when tumors reached an average volume of app. 40 mm³, **with test compound vehicle,** oral gavage, once a day, for 10 days and starting 72 h after vehicle virus injection.
3. **HSV-1 (quantity 1; 5×10⁶ pfu)-treated group: 50 µL of HSV-1** (5×10⁶), intratumoral injection when tumors reached an average volume of app. 40 mm³, **with test compound vehicle,** oral gavage, once a day, for 10 days and starting 72 h after virus injection, and **with anti-PD1/anti-CTLA4 vehicle,** intraperitoneal injection (200 µL PBS) on the same day as the virus injection, and then 3 administrations spaced 3 days apart each other (day 7, 10, 13 and 16).
4. **HSV-1 (quantity 2; 1×10⁸ pfu)-treated group: 100 µL of HSV-1** (1×10⁸) intratumoral injection when tumors reached an average volume of app. 40 mm³, **with test compound** vehicle, oral gavage, once a day, for 10 days and starting 72 h after virus injection.
5. **HSV-1 (quantity 1; 5×10⁶ pfu)-test compound-treated group: 50 µL of HSV-1** (5×10⁶), intratumoral injection when tumors reached an average volume of app. 40 mm³, **with Example 7(-)** (10 mg/kg, 20 mg/mL stock in DMSO, final 1 mg/mL, 5% DMSO; 0.5% HPMC in PBS (w/v), the suspension has been sonicated (2 min ultrasound bath, room temperature)), oral gavage (200 µL), once a day, for 10 days and starting 72 h after virus injection, and **with anti-PD1/anti-CTLA4 vehicle,** intraperitoneal injection (200 µL, PBS) on the same day as the virus injection, and then 3 administrations spaced 3 days apart each other (day 7, 10, 13 and 16).
6. **HSV-1 (quantity 1; 5×10⁶ pfu)-test compound-treated and anti-PD1** (anti-PD1) - (clone RMP1-14) / **anti-CTLA4** (anti-CTLA4) - (clone UC10-4F10-11) **treated-group: 50 µL of HSV-1** (5×10⁶) intratumoral injection when tumors reached an average volume of 40 mm³, **with Example 7(-)** (10 mg/kg), oral gavage, once a day, for 10 days and starting 72 h after virus injection, and **with anti-PD1/anti-CTLA4 antibody combination,** intraperitoneal injection (200 µL, 5 mg/kg, 1 mg/mL stock solution) on the same day as the virus injection and then 3 administrations spaced 3 days apart each other (day 7, 10, 13 and 16).
7. **HSV-1 (quantity 2; 1×10⁸ pfu)-test compound-treated group: 100 µL of HSV-1** (1×10⁸), intratumoral injection when tumors reached an average volume of app. 40 mm³, **with Example 7(-)** (10 mg/kg), oral gavage, once a day, for 10 days and starting 72 h after virus injection.
8. **Inactive HSV-1 (quantity 1; 5×10⁶ pfu)-test compound-treated group: 50 µL of HSV-1** (5×10⁶) intratumoral injection when tumors reached an average volume of 40 mm³, **with Example 7(-)** (10 mg/kg), oral gavage, once a day, for 13 days and starting immediately after virus injection.

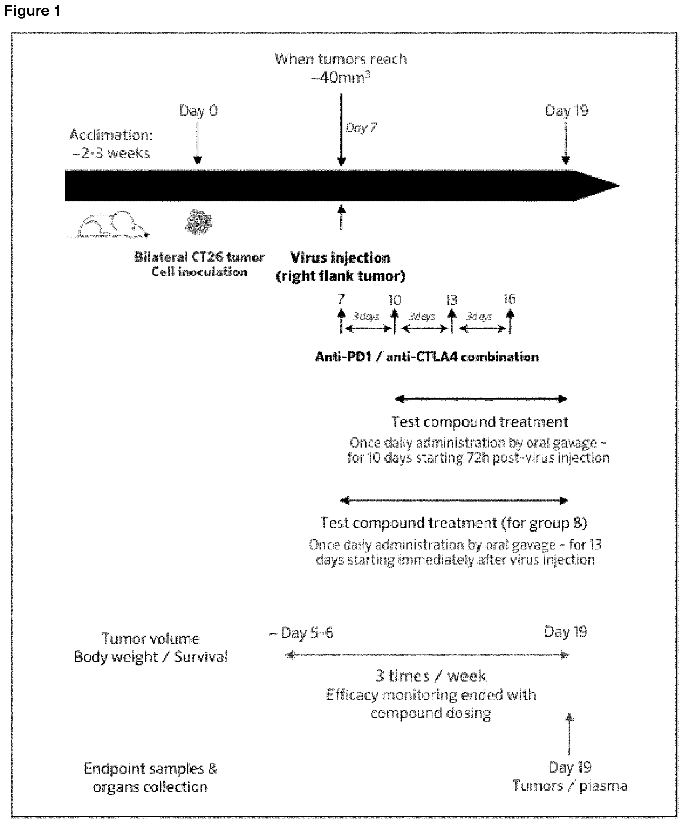

All the treatment and monitoring schedules were performed according to **Figure 1****.**

### Animal monitoring

As previously mentioned, tumor volume and body weight of the animals were measured and recorded three times per week. A tumor volume exceeding 2500 mm³ for one tumor or 3500 mm³ for both tumors, or a weight loss greater than 15% relative to the initial weight of the animal (done on day 6) were considered as an endpoint. Similarly, if the mouse was (i) prostrated, or (ii) no longer cleaned its coat (hair bristling and not glossy), (iii) was less mobile, this was also considered as an endpoint. When at least one of these conditions was met then the mice were sacrificed by cervical dislocation occurring in isolation from the other animals. All these behavioral and weight changes were recorded in a file that was accessible to staff of the animal facility (committee of animal welfare) and people directly involved in the study.

### Pain, procedures to reduce, avoid and mitigate any form of animal suffering

To minimize pain, suffering and anxiety related to the model, the animals were monitored regularly: every 2 days. The observation included reliable criteria such as weight loss and change of posture. An environmental enrichment was made to minimize anxiety. For surgical steps (subcutaneous injection of tumor cells), anesthesia was achieved using isoflurane (4% induction and 2% maintain).

### Fate of animals at the end of the experimental procedure

Animals which were beyond the limit points mentioned (see previous sections) were sacrificed by cervical dislocation. At the end of the study, remaining animals were processed for organ sampling and sacrificed.

### Anti-tumor response assessment

Starting from day 6 after bilateral CT26 tumor cell inoculation, all experimental animal groups, each consisting of 8 mice, were monitored 3 times per week over a period of ~2 weeks (from day 6 to day 19) for the following parameters:
- Tumor size: measured by physical examination, 3 times per week (for the first days post-tumor cell inoculation, follow up has been performed more closely so as not to miss the tumor volume average for virus injection);
- Body weight: monitored 3 times per week;
- Survival: 3 times per week, represented as a Kaplan-Meier plot.

### Endpoint sample & organ collection

### a. Early collection

At day 13 post-tumor cell inoculation, 4 mice from experimental group 4 (treated with the HSV-1 virus only at 1×10⁸ pfu) were processed for organ sampling and sacrificed. Mice were anesthetized using isoflurane gas anesthesia (4% induction and 2% maintain) and treated with an analgesic (buprenorphine, 0.1 mg/kg, subcutaneous injection) prior to cardiac puncture, for plasma sample collection. In addition, liver, brain, right and left tumors were collected, snap-frozen, and stored at -80°C until further analysis.

Other samples were collected on mice sacrificed before the end of the study so as to better understand the reason of the death. On day 13 post-tumor inoculation, one mouse from experimental group 6 (treated with HSV-1 virus - 5×10⁶ pfu, **Example 7(-)** 72 h post-virus injection and anti-PD1/anti-CTLA-4 antibody combination) had to be sacrificed due to body weight loss and the presence of an abscess in maxillary lymph nodes. A sample from this abscess was collected immediately after euthanasia by cervical dislocation, snap-frozen, and stored at -80°C until shipment to the sponsor for further analysis.

This type of abscess has also been noticed on one mouse from experimental group 8, treated with HSV-1 virus - 5×10⁶ inactivated as immediately followed by **Example 7(-)** administration. On day 16 post-tumor inoculation, this mouse was anesthetized using isoflurane gas anesthesia (5% induction) and blood was collected on heparin-coated tubes, by retro-orbital puncture. Plasma obtained was stored at -80°C until further analysis.

### b. Final collection

At the end of efficacy monitoring period (on day 19 post-tumor cell inoculation, around ~3-4 h after the last treatment with test compound), all surviving mice were anesthetized using isoflurane gas anesthesia (4% induction and 2% maintain) prior to blood sampling on heparin-coated tubes by retro-orbital puncture, for plasma collection. In addition, tumors were collected, snap-frozen, and stored at -80°C until further analysis.

### Results of the oHSV Experiment

Left and right tumor volumes increase from day 0 until day 7 in all animal groups (inoculation with CT-26 cells and non-treatment phase of the experiment).

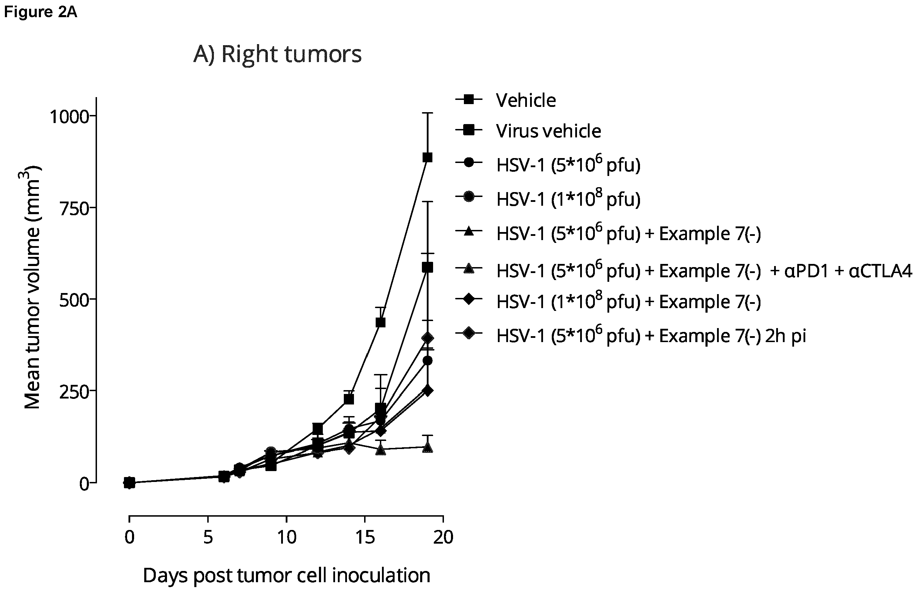

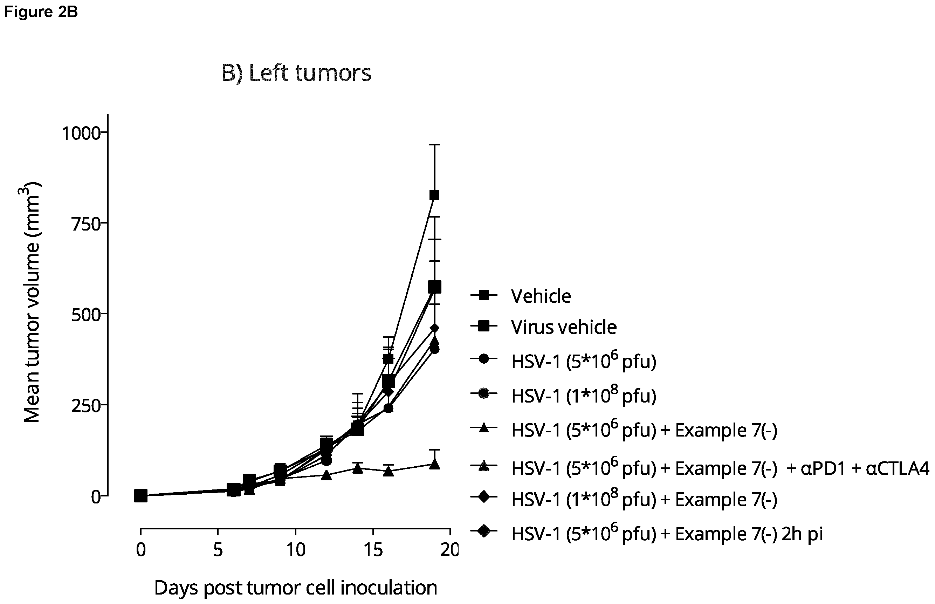

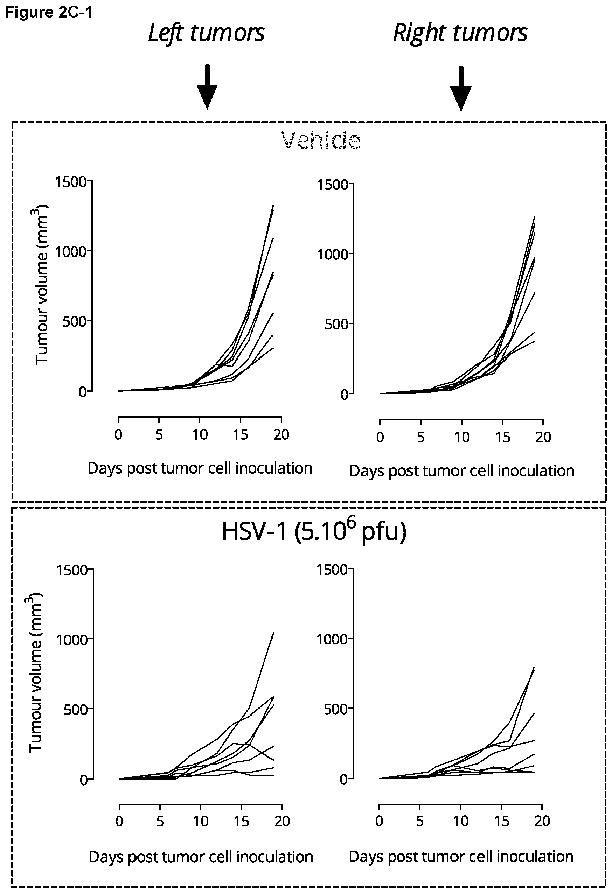

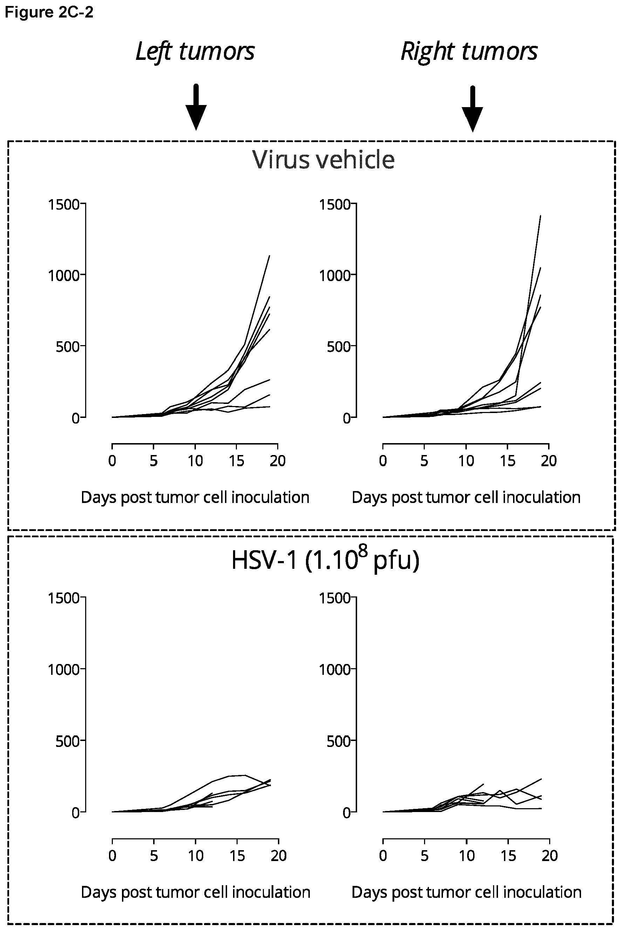

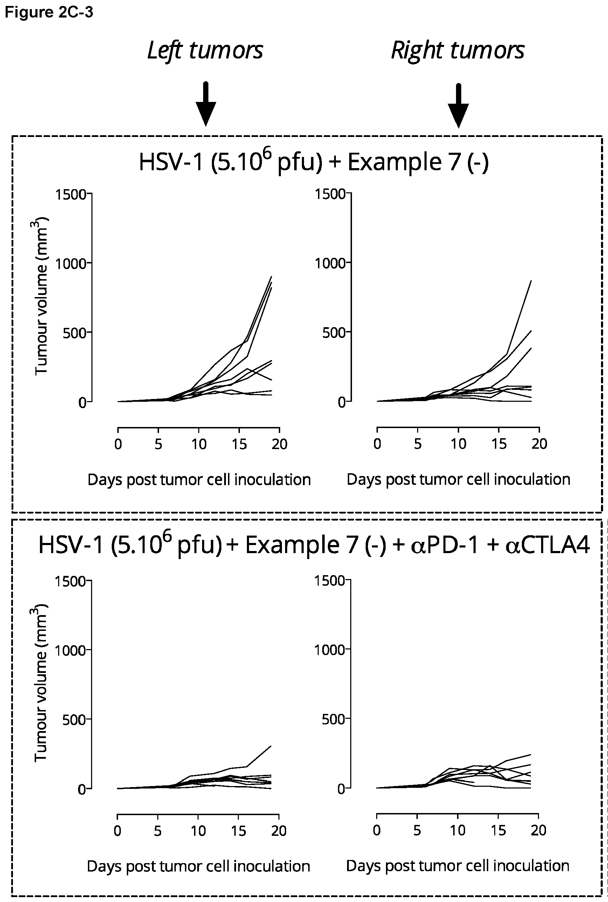

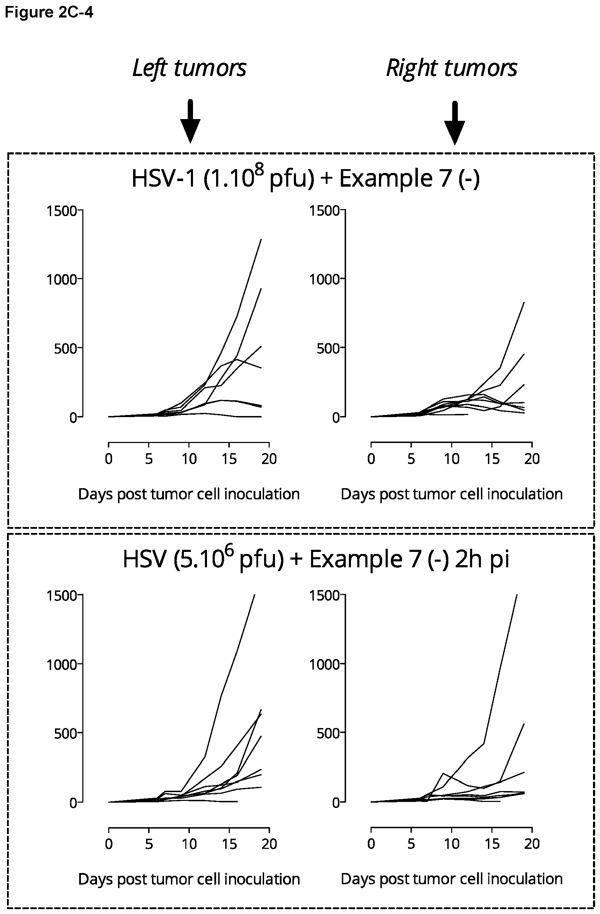

The highest tumor volume is measured in the vehicle group as expected on day 19. Vehicle injection into the right tumors leads to delayed tumor growth compared to vehicle **(****Figure 2****).** Figure 2A and 2B show median tumor volume (mm3) whereas Figure 2C monitors the tumor volume of individual mice over time.

Intratumoral injection of HSV virus reduces tumor growth (was able to cytolytically affect tumor cells) dose dependently on right flank treated tumors (Figure 2A) and left flank non-treated tumors (Figure 2B).

### Results of Figure 2A:

### Median tumor volume (mm³) - Right tumors

| Days | Vehicle | Virus vehicle | HSV-1 5.10⁶ | HSV-1 1.10⁸ | HSV-1 5.106 + Example 7(-) 72h pi | HSV-1 5.106 + Example 7(-) 72h pi + aPD-1 + aCTLA4 | HSV-1 1.108 + Example 7(-) 72h pi | HSV-1 5.106 + Example 7(-) 0h pi |
|---|---|---|---|---|---|---|---|---|
| 0 | 0 | 0 | 0 | 0 | 0 | 0 | 0 | 0 |
| 5 | 15,555 | 15,795 | 16,08 | 16,51 | 17,665 | 16,235 | 16,55 | 16,705 |
| 7 | 26,7 | 38,055 | 37,785 | 30,72 | 36,435 | 31,03 | 39,735 | 25,5 |
| 10 | 51,285 | 51,27 | 76,225 | 79,965 | 45,605 | 72,935 | 74,725 | 43,32 |
| 12 | 136,46 | 80,325 | 81,18 | 69,38 | 76,655 | 94,56 | 115,17 | 48,78 |
| 14 | 219,85 | 100,37 | 132,525 | | 86,87 | 105,02 | 142,49 | 39,01 |
| 17 | 442,445 | 136,43 | 145,135 | | 99,21 | 60,17 | 93,34 | 60,71 |
| 19 | 964,935 | 508,89 | 223,175 | | 105 | 84,68 | 100 | 72,69 |

### Results of Figure 2B:

### Median tumor volume (mm³) - Left tumors

| Days | Vehicle | Virus vehicle | HSV-1 5.10⁶ | HSV-1 1.10⁸ | HSV-1 5.106 + Example 7(-) 72h pi | HSV-1 5.106 + Example 7(-) 72h pi + aPD-1 + aCTLA4 | HSV-1 1.108 + Example 7(-) 72h pi | HSV-1 5.106 + Example 7(-) 0h pi |
|---|---|---|---|---|---|---|---|---|
| 0 | 0 | 0 | 0 | 0 | 0 | 0 | 0 | 0 |
| 5 | 16,355 | 13,935 | 14,56 | 11,175 | 14,155 | 14,66 | 18,17 | 15,755 |
| 7 | 24,52 | 40,155 | 28,805 | 20,19 | 22,85 | 18,32 | 23,55 | 21,61 |
| 10 | 42,84 | 67,015 | 62,22 | 39,77 | 55,18 | 47,345 | 33,055 | 43,385 |
| 12 | 152,19 | 132,27 | 118,445 | 87,655 | 121,82 | 58,12 | 92,98 | 73,31 |
| 14 | 199,29 | 208,155 | 169,485 | | 141,445 | 71,44 | 224,29 | 112,98 |
| 17 | 382,235 | 401,97 | 242,48 | | 219,98 | 66,86 | 348,78 | 169,68 |
| 19 | 835,52 | 669,575 | 380,81 | | 285,49 | 49,84 | 352,821 | 474,53 |

Higher intratumoral HSV injections lead to stronger reduction of tumor growth by day 19. However, the higher dose leads to death of 50% of the mice. When the high dose group of mice is subsequently treated with **Example 7(-)** the mice can be rescued and the reduction of tumor growth significantly prevails. Thus, higher viral doses can be applied and the oncolytic virus does not need to be attenuated for use as demonstrated with the clinical HSV isolate, since it can be controlled by the antiviral **Example 7(-).** Furthermore, the virus can not only be used topically but also systemically, since a general viraemia as described above can also be controlled by said antiviral compound due to its preferred pharmacokinetic profile and high brain exposure.

Inhibition or control of oncolytic herpesviruses with e.g. **Example 7(-)** was demonstrated by measuring the viral DNA within treated tumor samples. The tumors were collected as decribed above, snap frozen in liquid nitrogen and DNA of ~25 mg tumor samples was extracted with a QIAamp DNA Mini Kit (Cat. No. 51326) on a QlAcube machine. 10 µL of the 200 µL HSV DNA containing elution volumue was quantified with an Argene HSV R-gene kit (Ref.: 69-004B) and normalized to 1 gram tumor sample (see below).

| Group | Group | Total HSV-1 [copy/g tumor] mean |
|---|---|---|
| Group 1 | Vehicle | 0 |
| Group 2 | Virus Vehicle | 0 |
| Group 3 low dose virus | HSV-1 5×10⁶ pfu | 184678 |
| Group 4 high dose virus | HSV-1 1×10⁸ pfu | 789244 |
| Group 5 low dose virus + drug | HSV-1 5×10⁶ pfu + 10 mg/kg **Example 7(-)** 72 pi | 154493 |
| Group 6 low dose virus + drug + checkpoint Antibody | HSV-1 5×10⁶ pfu + 10 mg/kg **Example 7(-)** 72 pi + αPD1 + αCTLA-4 | 51301 |
| Group 7 high dose virus + drug | HSV-1 1×10⁸ pfu + 10 mg/kg **Example 7(-)** 72hpi | 74312 |
| Group 8 low dose virus + immediate drug treatment | HSV 5×10⁶ pfu + 10 mg/kg **Example 7(-)** 2h pi | 10600 |

The HSV-1 copy number per gram of tumor on day 19 (end of study) is highest in the untreated high dose virus group 4 and lower in low dose virus group 3. The viral load can be reduced with compound in groups 5, 6, 7 and 8. Treatment with compound e.g. **Example 7(-)** 2 hours post infection (pi) leads to the highest reduction in viral DNA load as compared to 72 hours delayed treatment after infection.

No virus was detected in uninfected groups 1 and 2.

Tumors of untreated dying mice of high dose virus group 4 had a mean HSV-1 copy/g of tumor of ~6×10⁹. This is at least 3 orders of magnitude higher than in the surviving animals of this group or compound treated groups to control viral replication.

A high viral dose is as efficacious as a low dose viral infection of the tumor, when the low dose oncolytic virus is combined with checkpoint inhibitors (Figure 2). Hence a combination of the optimal dose of the oncolytic virus can be controlled with the claimed compounds, may be combined with checkpoint inhibitors and used for tumors inside the experimental animal or patients in the clinic.

Finally, the triggered immune response not only leads to a reduction in tumor size of the oHSV injected right flank tumors (Figue 2A) but also to a reduction in size of the untreated tumor on the left flank (Figure 2B).

Rechallenge of the mice that cleared left and right flank tumors with CT-26 tumor cells leads to complete rejection of the CT-26 tumor cells or in other words no tumors can be grown on mice that cleared tumors indicating an adaptive immune response to CT-26 tumor cells.

In a second experiment a series of helicase primase inhibitors (**Example 10,** pritelivir and amenamevir) was analysed regarding efficacy in combination with repeated oncolytic virotherapy at constant dose in a syngeneic bilaterally-inoculated subcutaneous CT26 tumor-bearing mouse model to prove that this concept is generally applicable for the class of helicase primase inhibitors.

The methods of the first experiment were used and the experimental design was modified in the second experiment as follows:
1. The oncolytic herpesvirus was, in contrast to the first experiment, injected multiple times at a constant dose at distinct intervals.
2. In addition to **Example 7(-)** used in the first experiment, the efficacy of a series of helicase primase inhibitors was tested in the second experiment to enable the class of helicase primase inhibitors in general.

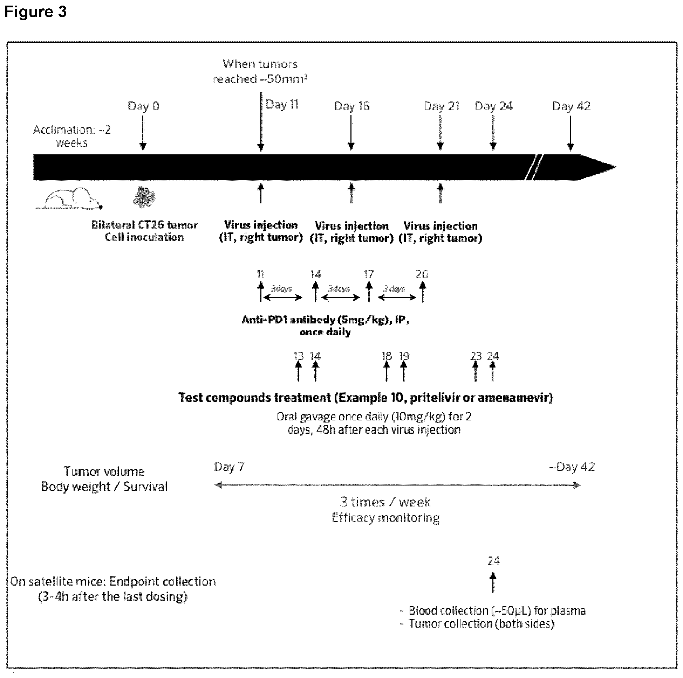

The outline of the second experiment is shown in Figure 3.

Pharmacological animal groups (10 animals per group, 80 animals in total and 5 animals per group in the satellite group; 50 animals in total used for analysis of plasma and tumor samples) were organized as such:
1. **Control virus-untreated group:** test compound vehicle, oral gavage, once a day, for 2 days and starting 48 h after each virus injection time-point.
2. **Control virus vehicle-treated group:** 100 µL of virus vehicle by intratumoral injection when tumors reached an average volume of ~50 mm³ (i.e. on day 11) then 2 injections spaced 5 days apart from each other; with test compound vehicle, oral gavage, once a day, for 2 days and starting 48 h after each virus injection time-point.
3. **HSV-1 (5×10⁷ pfu)-treated group:** 100 µL of HSV-1 (5×10⁷) by intratumoral injection when tumors reached an average volume of ~50 mm³ (i.e. on day 11) then 2 injections spaced 5 days apart from each other; with test compound vehicle, oral gavage, once a day, for 2 days and starting 48 h after each virus injection time-point and with anti-PD-1 vehicle, intraperitoneal injection (100 µL PBS) on the same day as the virus injection, and then 3 administrations spaced 3 days apart from each other (days 11, 14, 17 and 20).
4. **HSV-1 (5×10⁷ pfu) + anti-PD1-treated group:** 100 µL of HSV-1 (5×10⁷ pfu) intratumoral injection when tumors reached an average volume of ~50 mm³ (i.e. on day 11) then 2 injections spaced 5 days apart from each other; with test compound vehicle, oral gavage, once a day, for 2 days and starting 48 h after each virus injection time-point and with anti-PD-1 (5 mg/kg), intraperitoneal injection on the same day as the virus injection, and then 3 administrations spaced 3 days apart from each other (days 11, 14, 17 and 20).
5. **HSV-1 (5×10⁷ pfu)-Example 10-treated group:** 100 µL of HSV-1 (5×10⁷ pfu) intratumoral injection when tumors reached an average volume of ~50 mm³ (i.e. on day 11) then 2 injections spaced 5 days apart from each other; with **Example 10** (10 mg/kg), oral gavage, once a day, for 2 days and starting 48 h after each virus injection time-point (days 13, 14, 18, 19, 23 and 24).
6. **HSV-1 (5×10⁷ pfu)-pritelivir-treated group:** 100 µL of HSV-1 (5×10⁷ pfu) intratumoral injection when tumors reached an average volume of ~50 mm³ (i.e. on day 11) then 2 injections spaced 5 days apart from each other; with pritelivir (10 mg/kg), oral gavage, once a day, for 2 days and starting 48 h after each virus injection time-point (days 13, 14, 18, 19, 23 and 24).
7. **HSV-1 (5×10⁷ pfu)-amenamevir-treated group:** 100 µL of HSV-1 (5×10⁷ pfu) intratumoral injection when tumors reached an average volume of ~5 0mm³ (i.e. on day 11) then 2 injections spaced 5 days apart from each other; with amenamevir (10 mg/kg), oral gavage, once a day, for 2 days and starting 48 h after each virus injection time-point (days 13, 14, 18, 19, 23 and 24).
8. **HSV-1 (5×10⁷ pfu)-Example 10 + anti-PD1-treated group:** 100 µL of HSV-1 (5×10⁷ pfu) intratumoral injection when tumors reached an average volume of ~50 mm³ (i.e. on day 11) then 2 injections spaced 5 days apart from each other; with **Example 10** (10 mg/kg), oral gavage, once a day, for 2 days and starting 48 h after each virus injection time-point (days 13, 14, 18, 19, 23 and 24) and with anti-PD-1 (5 mg/kg), intraperitoneal injection on the same day as the virus injection and then 3 administrations spaced 3 days apart each other (days 11, 14, 17 and 20).

### Results of the second oHSV Experiment

Left and right tumor volumes increase from day 0 until day 7 in all animal groups (inoculation with CT-26 cells and non-treatment phase of the experiment).

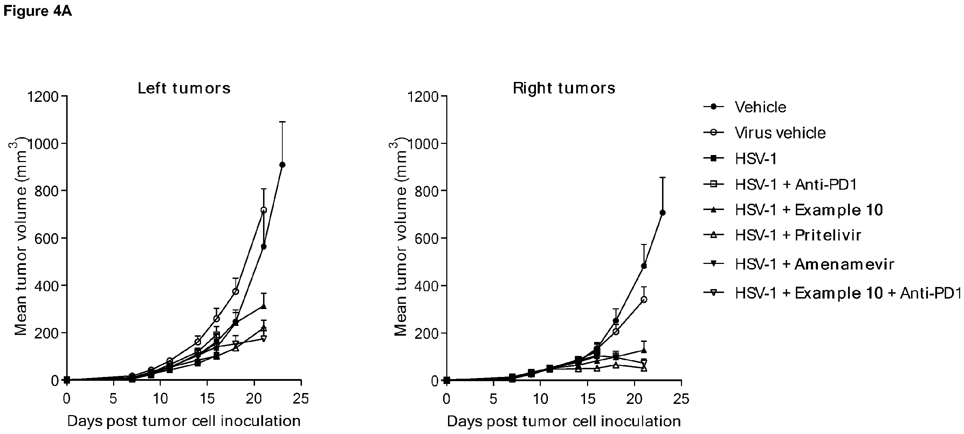

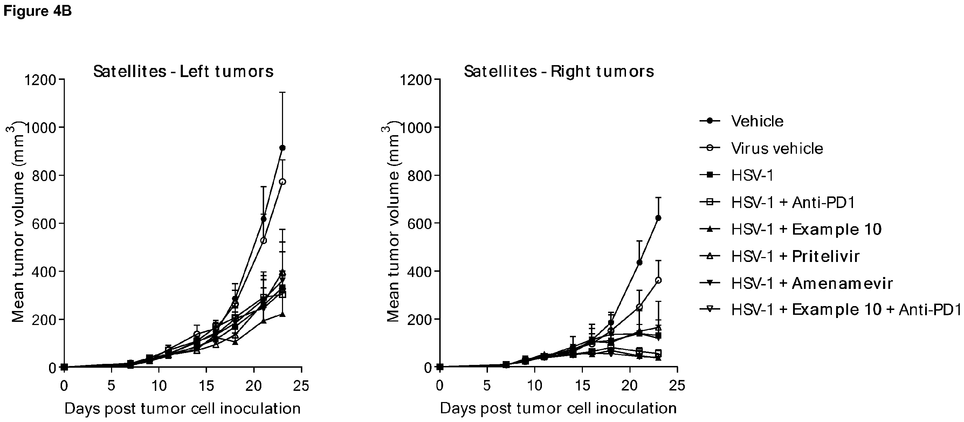

The highest tumor volume is measured in the vehicle group as expected on day 19. Vehicle injection into the right tumors leads to delayed tumor growth compared to vehicle (Figure 4A and 4B satellite animals).

Intratumoral injection of HSV virus reduces tumor growth (i.e. intratumoral injection of HSV virus was able to cytolytically affect tumor cells) dose dependently on right flank treated tumors in the first experiment (Figure 2A) and left flank non-treated tumors (Figure 2B). Multiple intratumoral injections of HSV virus in the second experiment (Figure 4A and B) reduces tumors growth as efficiently as a single high dose virus injection in the first experiment (Figure 2A and B).

Higher intratumoral HSV injections (first experiment) or multiple intratumoral HSV injection in the second experiment lead to stronger reduction of tumor growth by day 19 or 21, respectively. However, the higher or multiple lower HSV doses lead to death of a significant number of the mice. When the high dose group of mice is subsequently treated with **Example 7(-)** in the first experiment or a series of other helicase primase inhibitors **(Example 10,** pritelivir or amenamevir) in the second experiment, the mice can be rescued and the reduction of tumor growth significantly prevails. Thus, higher or multiple viral does can be applied and the oncolytic virus does not need to be attenuated for use as demonstrated with the clinical HSV isolate, since it can be controlled by the antiviral **Example 7(-)** or helicase primase inhibitors in general.

Inhibition or control of oncolytic herpesviruses with e.g. **Example 7(-)** was demonstrated by measuring the viral DNA within treated tumor samples. Survial of mice was analysed on day 21 in the second experiment. Treatment of mice with helicase primase inhibitors significantly rescue mice from death (see table below, number of dead animals by day) while the anti-tumor effect of the oncolytic virus prevails (Figure 4).

### Number of dead animals by day:

| Day | Vehicle | Virus vehicle | HSV-1 | HSV-1 anti-PD-1 | HSV-1 **Example 10** | HSV-1 pritelivir | HSV-1 amenamevir | HSV-1 anti-PD-1 + **Example 10** |
|---|---|---|---|---|---|---|---|---|
| 7 | 0 | 0 | 0 | 0 | 0 | 0 | 0 | 0 |
| 9 | 0 | 0 | 0 | 0 | 0 | 0 | 0 | 0 |
| **11** | 0 | 0 | 0 | 0 | 0 | 0 | 0 | 0 |
| 14 | 0 | 0 | 0 | 0 | 0 | 0 | 0 | 0 |
| **16** | 0 | 0 | 0 | 1 | 0 | 0 | 0 | 0 |
| 18 | 0 | 0 | 3 | 5 | 1 | 0 | 2 | 1 |
| **21** | 0 | 0 | 5 | 6 | 1 | 0 | 2 | 1 |

Analysis of the viral load as described above for the first experiment revealed high HSV copy numbers in oncolytic virus treated right flank tumors and absence of HSV DNA in 10 randomly selected plasma samples and left flank tumors which implies that the anti-tumor effect on the non-oncolytic treated left flank tumor is mediated most likely by the stimulated immunse system (data not shown).

### DESCRIPTION OF THE FIGURES

**Figure 1****:** Treatment and monitoring schedules of the oHSV animal model experiment.
**Figure 2A** **and** **2B****:** Mean tumor volume (mm³) of CT26 bearing mice (bilaterally inoculated) treated with vehicle only (n=8), virus vehicle (n=8), HSV-1 virus - 5×10⁶ (n=8), HSV-1 virus - 1×10⁸ (n=8), HSV-1 virus - 5×10⁶ and **Example 7(-)** 72 h pi combination (n=8), HSV-1 virus - 5*10⁶ and **Example 7(-)** 72 h pi and anti-PD1/anti-CTLA4 mAb combination (n=8), HSV-1 virus - 1×10⁸ and **Example 7(-)** 72 h pi combination (n=8), HSV-1 virus - 5×10⁶ inactivated immediately with **Example 7(-)** (n=8). Data are presented for **right flank tumors (A** - **upper panel)** and **left flank tumors (B** - **lower panel)** as mean ± SEM. NB: **Mean was not calculated if more than one animal in the experimental group was sacrificed.**
Figure 2C: Individual tumor volume (mm³) of **left and right flank tumors** CT26 bearing mice (bilaterally inoculated) treated vehicle only (n=8), virus vehicle (n=8), HSV-1 virus - 5×10⁶ pfu (n=8), HSV-1 virus - 1×10⁸ pfu (n=8), HSV-1 virus - 5×10⁶ pfu and **Example 7(-)** combination (n=8), HSV-1 virus - 5×10⁶ pfu and **Example 7(-)** and anti-PD1/anti-CTLA4 antibodies combination (n=8), HSV-1 virus - 1×10⁸ pfu and **Example 7(-)** combination (n=8), HSV-1 virus - 5×10⁶ pfu inactivated 2 hours after with **Example 7(-)** (n=8).
**Figure 3****:** Treatment and monitoring schedule of the second oHSV animal experiment.
**Figure 4A****:** Mean tumor volume (mm³) of CT26 bearing mice (bilaterally inoculated) **efficacy mice** treated either with oral gavage vehicle only (n=10), virus vehicle (n=10), HSV-1 virus - 5×10⁷ pfu (n=10), HSV-1 virus - 5×10⁷ pfu and anti-PD1 combination (n=10), HSV-1 virus - 5×10⁷ pfu and **Example 10** combination (n=10), HSV-1 virus - 5×10⁷ pfu and pritelivir combination (n=10), HSV-1 virus - 5×10⁷ pfu and amenamevir combination (n=10), or HSV-1 virus - 5×10⁷ pfu + **Example 10** and anti-PD1 combination (n=10). Data are presented for **left flank tumors (left panel)** and **right flank tumors (right panel)** as mean ± SEM.
**Figure 4B****:** Mean tumor volume (mm³) of CT26 bearing mice (bilaterally inoculated) **satellite mice** treated either with oral gavage vehicle only (n=5), virus vehicle (n=5), HSV-1 virus - 5×10⁷ pfu (n=5), HSV-1 virus - 5×10⁷ pfu and anti-PD1 combination (n=5), HSV-1 virus - 5×10⁷ pfu and **Example 10** combination (n=5), HSV-1 virus - 5×10⁷ pfu and pritelivir combination (n=5), HSV-1 virus - 5×10⁷ pfu and amenamevir combination (n=5), or HSV-1 virus - 5×10⁷ pfu + **Example 10** and anti-PD1 combination (n=5). Data are presented for **left flank tumors (left panel)** and **right flank tumors (right panel)** as mean ± SEM.

## Claims

1. Helicase primase inhibitors for the use in a combination therapy with oncolytic herpesviruses for treating cancer, wherein the helicase primase inhibitors are selected from compounds having a structure of the Formula (I) 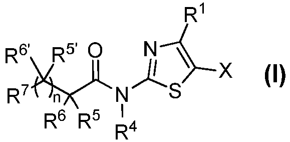 an enantiomer, diastereomer, tautomer, *N*-oxide, solvate, formulation or pharmaceutically acceptable salt thereof, wherein
X is selected from 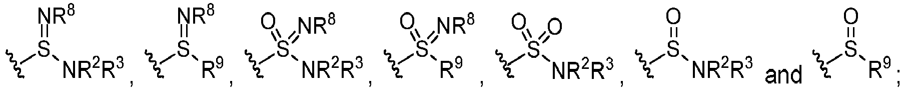
R¹ is selected from H, halogen, C₁₋₆-alkyl, halo-C₁₋₆-alkyl, hydroxy-C₁₋₆-alkyl, C₃₋₆-cycloalkyl, halo-C₃₋₆-cycloalkyl, -O-C₁₋₆-alkyl, -O-halo-C₁₋₆-alkyl and -NH-C₁₋₆-alkyl;
R² is selected from H, -CN, -NO₂, C₁₋₁₀-alkyl, C₂₋₁₀-alkenyl, C₂₋₁₀-alkynyl, C₀₋₁₀-alkylene-C₃₋₁₀-cycloalkyl, C₀₋₁₀-alkylene-C₃₋₁₀-heterocycloalkyl, C₀₋₁₀-alkylene-(5- to 10-membered heteroaryl), C₀₋₁₀-alkylene-(6- to 10-membered aryl), C₀₋₁₀-alkylene-(6- to 10-membered heteroaryl), C₀₋₁₀-alkylene-OR¹¹, C₀₋₁₀-alkylene-CO₂R¹¹, C₀₋₁₀-alkylene-C(=O)NR¹¹R¹², C₀₋₁₀-alkylene-C(=S)NR¹¹R¹², C₀₋₁₀-alkylene-C(=O)NR¹¹SO₂R¹³, C₀₋₁₀-alkylene-C(=S)NR¹¹SO₂R¹¹, C₀₋₁₀-alkylene-C(=O)R¹¹, C₀₋₁₀-alkylene-C(=S)R¹¹, C₀₋₁₀-alkylene-SR¹¹, C₀₋₁₀-alkylene-SOₓR¹³, C₀₋₁₀-alkylene-SO₃R¹¹, C₀₋₁₀-alkylene-SO₂NR¹¹R¹², C₀₋₁₀-alkylene-NR¹¹C(=O)R¹¹, C₀₋₁₀-alkylene-NR¹¹C(=S)R¹¹, C₀₋₁₀-alkylene-NR¹¹SO₂R¹³, C₀₋₁₀-alkylene-NR¹¹C(=O)NR¹¹R¹², C₀₋₁₀-alkylene-NR¹¹C(=S)NR¹¹R¹², C₀₋₁₀-alkylene-NR¹¹SO₂NR¹¹R¹², C₀₋₁₀-alkylene-NR¹¹R¹², wherein alkyl, alkenyl, alkynyl, alkylene, cycloalkyl, heterocycloalkyl, aryl and heteroaryl is unsubstituted or substituted with 1 to 7 substituents independently selected from the group consisting of oxo, CN, -NO₂, OR¹¹, O-C₂₋₆-alkylene-OR¹¹, C₁₋₆-alkyl, halo-C₁₋₆-alkyl, halogen, CO₂R¹¹, C(=O)NR¹¹R¹², C(=O)NR¹¹SO₂R¹¹, C(=O)R¹¹, SR¹¹, SOₓR¹¹, SO₃R¹¹, P(=O)(OR¹¹)₂, SO₂NR¹¹R¹², NR¹¹C(=O)R¹¹, NR¹¹SO₂R¹³, NR¹¹C(=O)NR¹¹R¹², NR¹¹SO₂NR¹¹R¹², C₃₋₁₀-cycloalkyl, O-C₃₋₁₀-cycloalkyl, C₃₋₁₀-heterocycloalkyl, O-C₃₋₁₀-heterocycloalkyl and NR¹¹R¹²;
R³ is selected from H, C₁₋₆-alkyl, halo-C₁₋₆-alkyl, -O-C₁₋₆-alkyl, -O-halo-C₁₋₆-alkyl, C₃₋₆-cycloalkyl and C₃₋₆-heterocycloalkyl, wherein alkyl, cycloalkyl and heterocycloalkyl are optionally substituted with 1 to 5 substituents independently selected from halogen, -CN, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
or R² and R³ when taken together with the nitrogen to which they are attached complete a 3- to 8-membered ring containing carbon atoms and optionally containing 1 or 2 heteroatoms selected from O, S or N, wherein the ring is unsubstituted or substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
R⁴ is selected from H, C₁₋₆-alkyl, C₁₋₆-acyl, C₂₋₆-alkenyl, C₃₋₈-cycloalkyl and C₃₋₈-heterocycloalkyl, wherein alkyl, acyl, alkenyl, cycloalkyl and heterocycloalkyl are optionally substituted with 1 to 5 substituents independently selected from halogen, -CN, OH, oxo, C₁₋₃-alkyl, halo-C₁₋₃-alkyl, O-C₁₋₃-alkyl and O-halo-C₁₋₃-alkyl;
R⁵ and R⁶ and R⁵' and R⁶' are independently selected from H, halogen, C₁₋₆-alkyl, NH₂, NHC₁₋₆-alkyl, N(C₁₋₆-alkyl)₂ and C₀₋₆-alkylene-C(=O)NH₂;
or R⁵ and R⁶ and R⁵' and R⁶' independently when taken together with the carbon to which they are attached complete a 3- to 8-membered ring containing carbon atoms and optionally containing 1 or 2 heteroatoms selected from O, S or N, wherein the ring is unsubstituted or substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
or R⁵ and R⁵' and R⁶ and R⁶' independently when taken together with the two adjacent carbon to which they are attached complete a 3- to 8-membered ring containing carbon atoms and optionally containing 1 or 2 heteroatoms selected from O, S or N, wherein the ring is unsubstituted or substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
or anyone of R⁵, R⁵', R⁶ and R⁶' together with R⁷ may form an 8- to 11-membered bicyclic ring, which is optionally substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
R⁷ is selected from a 6-membered aryl and 5- or 6-membered heteroaryl, wherein aryl and heteroaryl are optionally substituted with 1 to 4 substituents independently selected from halogen, -CN, -NO₂, OH, C₁₋₆-alkyl, O-C₁₋₆-alkyl, C₃₋₆-cycloalkyl, O-C₃₋₆-cycloalkyl, C₃₋₆-heterocycloalkyl, O-C₃₋₆-heterocycloalkyl, SO_{y-}C₁₋₆-alkyl, CO₂H, C(=O)O-C₁₋₆-alkyl, 6- to 10-membered aryl, 5- to 10-membered heteroaryl, O-(6- to 10-membered aryl) and O-(5- to 10-membered heteroaryl), wherein alkyl, cycloalkyl, heterocycloalkyl, aryl and heteroaryl are optionally substituted with 1 to 5 substituents independently selected from halogen, -CN, - NO₂, OH, R¹³, OR¹³, CO₂R¹¹, NR¹¹R¹², C(=O)R¹¹, C(=S)R¹¹, C(=O)NR¹¹R¹², NR¹¹C(=O)NR¹¹R¹², NR¹¹C(=O)OR¹³, OC(=O)NR¹¹R¹², C(=S)NR¹¹R¹², NR¹¹C(=S)NR¹¹R¹², NR¹¹C(=S)OR¹³, OC(=S)NR¹¹R¹²; SO_{y}-C₁₋₆-alkyl, SO_{y}-halo-C₁₋₆-alkyl, SR¹¹, SOₓR¹³, SO₃R¹¹, SO₂NR¹¹R¹², NR¹¹SO₂R¹³ and NR¹¹SO₂NR¹¹R¹²;
R⁸ is selected from H, -CN, -NO₂, C₁₋₁₀-alkyl, C₂₋₁₀-alkenyl, C₂₋₁₀-alkynyl, C₀₋₁₀-alkylene-C₃₋₁₀-cycloalkyl, C₀₋₁₀-alkylene-C₃₋₁₀-heterocycloalkyl, C₀₋₁₀-alkylene-(5- to 10-membered heteroaryl), C₀₋₁₀-alkylene-(6- to 10-membered aryl), C₀₋₁₀-alkylene-(6- to 10-membered heteroaryl), C₀₋₁₀-alkylene-OR¹¹, C₀₋₁₀-alkylene-CO₂R¹¹, C₀₋₁₀-alkylene-C(=O)NR¹¹R¹², C₀₋₁₀-alkylene-C(=S)NR¹¹R¹², C₀₋₁₀-alkylene-C(=O)NR¹¹SO₂R¹³, C₀₋₁₀-alkylene-C(=S)NR¹¹SO₂R¹¹, C₀₋₁₀-alkylene-C(=O)R¹¹, C₀₋₁₀-alkylene-C(=S)R¹¹, C₀₋₁₀-alkylene-SR¹¹, C₀₋₁₀-alkylene-SOₓ-R¹³, C₀₋₁₀-alkylene-SO₃R¹¹, C₀₋₁₀-alkylene-SO₂NR¹¹R¹², C₀₋₁₀-alkylene-NR¹¹C(=O)R¹¹, C₀₋₁₀-alkylene-NR¹¹C(=S)R¹¹, C₀₋₁₀-alkylene-NR¹¹SO₂R¹¹, C₀₋₁₀-alkylene-NR¹¹C(=O)NR¹¹R¹², C₀₋₁₀-alkylene-NR¹¹C(=S)NR¹¹R¹², C₀₋₁₀-alkylene-NR¹¹-SO₂-NR¹¹R¹², C₀₋₁₀-alkylene-NR¹¹R¹², wherein alkyl, alkenyl, alkynyl, alkylene, cycloalkyl, heterocycloalkyl, aryl and heteroaryl is unsubstituted or substituted with 1 to 7 substituents independently selected from the group consisting of oxo, CN, -NO₂, OR¹¹, O-C₂₋₆-alkylene-OR¹¹, C₁₋₆-alkyl, halo-C₁₋₆-alkyl, halogen, CO₂R¹¹, CONR¹¹R¹², CONR¹¹SO₂R¹¹, COR¹¹, SOₓR¹¹, SO₃H, PO(OH)₂, SO₂NR¹¹R¹², NR¹¹COR¹¹, NR¹¹SO₂R¹¹, NR¹¹-CO-NR¹¹R¹², NR¹¹-SO₂-NR¹¹R¹², C₃₋₁₀-cycloalkyl, O-C₃₋₁₀-cycloalkyl, C₃₋₁₀-heterocycloalkyl, O-C₃₋₁₀-heterocycloalkyl and NR¹¹R¹²;
R⁹ is selected from C₁₋₁₀-alkyl, C₂₋₁₀-alkenyl, C₂₋₁₀-alkynyl, C₀₋₁₀-alkylene-C₃₋₁₀-cycloalkyl, C₀₋₁₀-alkylene-C₃₋₁₀-heterocycloalkyl, C₀₋₁₀-alkylene-(5- to 10-membered heteroaryl), C₀₋₁₀-alkylene-(6- to 10-membered aryl), C₀₋₁₀-alkylene-(6- to 10-membered heteroaryl), C₀₋₁₀-alkylene-OR¹¹, C₀₋₁₀-alkylene-CO₂R¹¹, C₀₋₁₀-alkylene-C(=O)NR¹¹R¹², C₀₋₁₀-alkylene-C(=S)NR¹¹R¹², C₀₋₁₀-alkylene-C(=O)NR¹¹SO₂R¹³, C₀₋₁₀-alkylene-C(=S)NR¹¹SO₂R¹¹, C₀₋₁₀-alkylene-C(=O)R¹¹, C₀₋₁₀-alkylene-C(=S)R¹¹, C₀₋₁₀-alkylene-SR¹¹, C₀₋₁₀-alkylene-SOₓR¹³, C₀₋₁₀-alkylene-SO₃R¹¹, C₀₋₁₀-alkylene-SO₂NR¹¹R¹², C₀₋₁₀-alkylene-NR¹¹C(=O)R¹¹, C₀₋₁₀-alkylene-NR¹¹C(=S)R¹¹, C₀₋₁₀-alkylene-NR¹¹SO₂R¹³, C₀₋₁₀-alkylene-NR¹¹C(=O)NR¹¹R¹², C₀₋₁₀-alkylene-NR¹¹C(=S)NR¹¹R¹², C₀₋₁₀-alkylene-NR¹¹SO₂NR¹¹R¹², C₀₋₁₀-alkylene-NR¹¹R¹², wherein alkyl, alkenyl, alkynyl, alkylene, cycloalkyl, heterocycloalkyl, aryl and heteroaryl is unsubstituted or substituted with 1 to 7 substituents independently selected from the group consisting of oxo, CN, -NO₂, OR¹¹, O-C₂₋₆-alkylene-OR¹¹, C₁₋₆-alkyl, halo-C₁₋₆-alkyl, halogen, CO₂R¹¹, C(=O)NR¹¹R¹², C(=O)NR¹¹SO₂R¹¹, C(=O)R¹¹, SR¹¹, SOₓR¹¹, SO₃R¹¹, P(=O)(OR¹¹)₂, SO₂NR¹¹R¹², NR¹¹C(=O)R¹¹, NR¹¹SO₂R¹³, NR¹¹C(=O)NR¹¹R¹², NR¹¹SO₂NR¹¹R¹², C₃₋₁₀-cycloalkyl, O-C₃₋₁₀-cycloalkyl, C₃₋₁₀-heterocycloalkyl, O-C₃₋₁₀-heterocycloalkyl and NR¹¹R¹²;
R¹¹ is independently selected from H, C₁₋₆-alkyl, C₀₋₆-alkylene-C₃₋₁₀-cycloalkyl and C₀₋₆-alkylene-C₃₋₁₀-heterocycloalkyl, wherein alkyl, alkylene, cycloalkyl and heterocycloalkyl is unsubstituted or substituted with 1 to 6 substituents independently selected from the group consisting of halogen, -CN, OH, oxo, C₁₋₃-alkyl, halo-C₁₋₃-alkyl, O-C₁₋₃-alkyl, O-halo-C₁₋₃-alkyl, NH₂, NH(C₁₋₃-alkyl), N(C₁₋₃-alkyl)₂, C₃₋₆-heterocycloalkyl, C₃₋₆-cycloalkyl, SO₂-NHC₁₋₃-alkyl, SO₂-N(C₁₋₃-alkyl)₂ and SO₂-C₁₋₃-alkyl, wherein cycloalkyl and heterocycloalkyl is unsubstituted or substituted with 1 to 3 substituents independently selected from the group consisting of F, OH, oxo, CH₃, CHF₂ and CF₃;
R¹² is independently selected from H, C₁₋₆-alkyl, halo-C₁₋₆-alkyl and C₃₋₆-cycloalkyl;
or R¹¹ and R¹² when taken together with the nitrogen to which they are attached complete a 3-to 8-membered ring containing carbon atoms and optionally containing 1 or 2 heteroatoms selected from O, S or N, wherein the ring is unsubstituted or substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
R¹³ is independently selected from C₁₋₆-alkyl, C₀₋₆-alkylene-C₃₋₁₀-cycloalkyl and C₀₋₆-alkylene-C₃₋₁₀-heterocycloalkyl, wherein alkyl, alkylene, cycloalkyl and heterocycloalkyl is unsubstituted or substituted with 1 to 6 substituents independently selected from the group consisting of halogen, -CN, OH, oxo, C₁₋₃-alkyl, halo-C₁₋₃-alkyl, O-C₁₋₃-alkyl, O-halo-C₁₋₃-alkyl, NH₂, NH(C₁₋₃-alkyl), N(C₁₋₃-alkyl)₂, C₃₋₆-heterocycloalkyl, C₃₋₆-cycloalkyl, SO₂-NHC₁₋₃-alkyl, SO₂-N(C₁₋₃-alkyl)₂ and SO₂-C₁₋₃-alkyl, wherein cycloalkyl and heterocycloalkyl is unsubstituted or substituted with 1 to 3 substituents independently selected from the group consisting of F, OH, oxo, CH₃, CHF₂ and CF₃;
n is selected from 0 and 1;
x is independently selected from 1 and 2;
y is independently selected from 0, 1 and 2;
and wherein optionally R¹ is connected to one residue selected from R², R³, R⁸, R⁹ or R¹¹ to form a 5- to 8-membered heterocycle, which is optionally substituted with 1 to 4 substituents independently selected from the group consisting of halogen, -CN, -NO₂, OH, oxo, C₁₋₃-alkyl, halo-C₁₋₃-alkyl, O-C₁₋₃-alkyl, O-halo-C₁₋₃-alkyl, SO₂-C₁₋₃-alkyl and CO₂H;
or having a structure of the following Formula 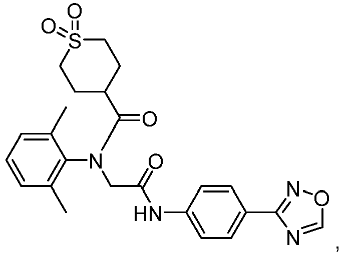
or a solvate, formulation or pharmaceutically acceptable salt thereof.

2. Helicase primase inhibitors for the use according to claim 1, having a structure of the Formula (Ia) or (Ib) 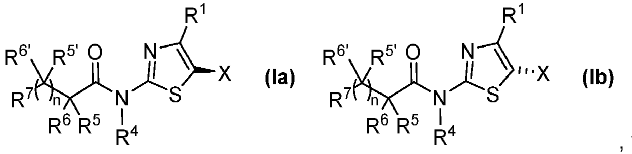 wherein
in the Formula (Ia) X is 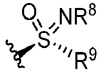 and in the Formula (Ib) X is 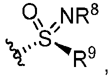 or a solvate, formulation or pharmaceutically acceptable salt thereof.

3. Helicase primase inhibitors for the use according to claim 1 or 2, which are represented by the Formula 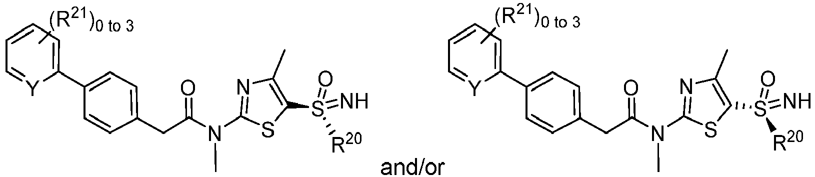 wherein
R²⁰ is selected from C₁₋₄-alkyl and C₃₋₆-cycloalkyl, wherein alkyl and cycloalkyl is unsubstituted or substituted with 1 to 3 substituents independently selected from the group consisting of F or Me;
R²¹ is selected from F, Cl, OH, Me, OMe, CHF₂, CF₃, OCHF₂, OCF₃; and
Y is selected from nitrogen or carbon,
or a solvate, formulation or pharmaceutically acceptable salt thereof.

4. Helicase primase inhibitors for the use according to anyone of claims 1 to 3, which are selected from the group consisting of 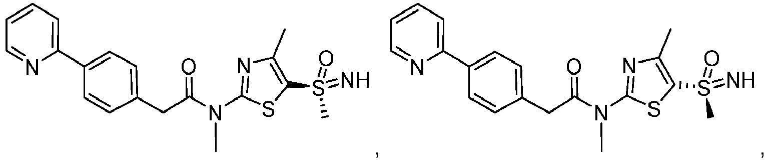 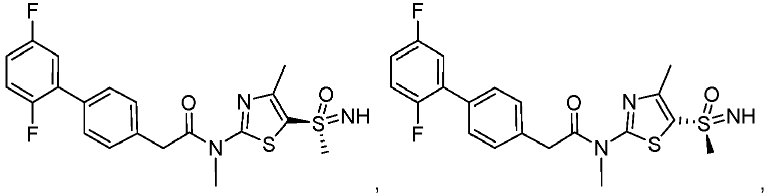 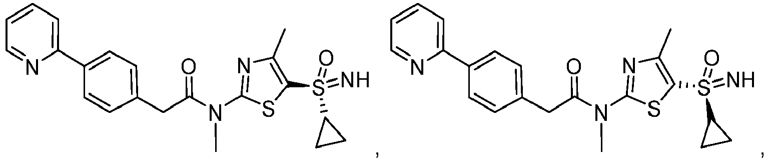 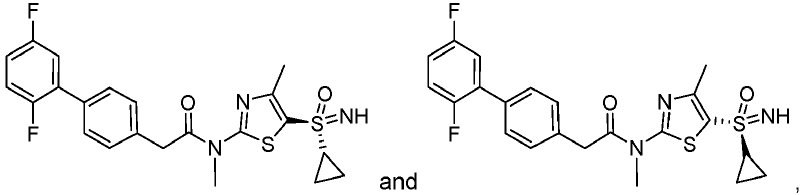 or a solvate, formulation or pharmaceutically acceptable salt thereof.

5. Helicase primase inhibitors for the use according to anyone of claims 1 to 4, having a structure selected from 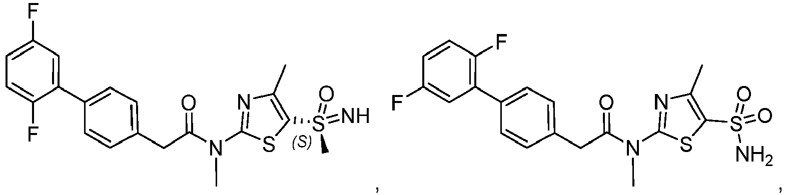 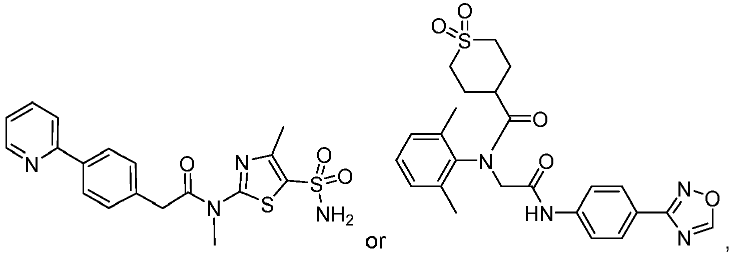 or a solvate, formulation or pharmaceutically acceptable salt thereof.

6. Helicase primase inhibitor for the use according to anyone of claims 1 to 5, which is 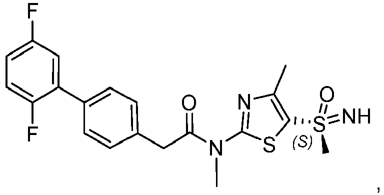 or a solvate, formulation or pharmaceutically acceptable salt thereof.

7. A pharmaceutical composition for the use as an antidote in a combination therapy with oncolytic herpesviruses for treating cancer, which comprises at least one helicase primase inhibitor as defined in any one of the preceding claims, which acts to control, modulate, inhibit or shut off the activity of oncolytic herpesviruses sensitive to said inhibitors used in cancer therapy, and which may further comprise at least one pharmaceutically acceptable carrier and/or excipient and/or at least one further active substance, such as antiviral active or immune modulating compounds, including checkpoint inhibitors, being effective in treating a disease or disorder associated with oncolytic viral infections used in the treatment of cancer.

8. The helicase primase inhibitors or the pharmaceutical composition for the use according to anyone of the preceding claims, wherein the cancer to be treated is solid cancer, preferably the cancer disease is selected from liver cancer, lung cancer, colon cancer, pancreas cancer, kidney cancer, brain cancer, melanoma and glioblastoma etc..

9. The helicase primase inhibitors or the pharmaceutical composition for the use according to anyone of the preceding claims, wherein the cancer therapy comprises infusion, injection, intratumoral injection or topical or transdermal application of the oncolytic herpesviruses or oncolytic herpesvirus infected cells and/or of the helicase primase inhibitors or the pharmaceutical composition comprising the same.

10. The helicase primase inhibitors or the pharmaceutical composition for the use according to anyone of the preceding claims, wherein the oncolytic herpesviruses or oncolytic herpesviruses infected cells are selected from an oncolytic wildtype, a clinical isolate or a laboratory herpesvirus strain or a genetically engineered or multi-mutated optionally attenuated or boosted oncolytic herpesvirus.

11. A kit comprising at least one of the helicase primase inhibitors or the pharmaceutical composition as defined in any one of the preceding claims and at least one oncolytic herpesvirus selected from a wildtype, a laboratory strain, a clinical isolate and a genetically engineered or multi-mutated oncolytic herpesvirus.

12. The kit according to claim 11 for the use in the treatment of cancer as defined in any one of the preceding claims.

13. The helicase primase inhibitors or the pharmaceutical composition as defined in any one of the preceding claims for use in a combination therapy with oncolytic herpesviruses for the treatment and prophylaxis of oncolytic herpes infections in cancer patients potentially with a suppressed immune system, such as AIDS patients, patients having a genetic or inherited immunodeficiency, transplant patients; in new-born children and infants; in herpes-positive patients, in particular oncolytic herpes-simplex-positive patients, for suppressing recurrence or oncolytic viral shedding; patients, in particular in herpes-positive patients, in particular oncolytic herpes-simplex-positive patients, who are resistant to nucleosidic antiviral therapy such as acyclovir, penciclovir, famciclovir, ganciclovir, valacyclovir and/or foscarnet or cidofovir.

## Patentansprüche

1. Helicase-Primase-Inhibitoren zur Verwendung in einer Kombinationstherapie mit onkolytischen Herpesviren zur Behandlung von Krebs, wobei die Helicase-Primase-Inhibitoren ausgewählt sind aus Verbindungen mit einer Struktur der Formel (I) 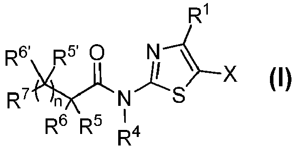 einem Enantiomer, Diastereomer, Tautomer, *N-Oxid,* Solvat, einer Formulierung oder einem pharmazeutisch akzeptablen Salz davon, wobei
X ausgewählt ist aus 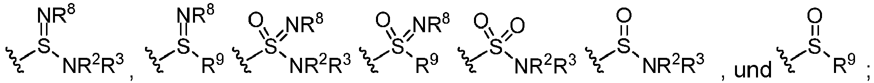
R¹ ausgewählt ist aus H, Halogen, C₁₋₆-Alkyl, Halogen-C₁₋₆-Alkyl, Hydroxy-C₁₋₆-Alkyl, C₃₋₆-Cycloalkyl, Halogen-C₃₋₆-cycloalkyl, -O-C₁₋₆-Alkyl, -O-Halogen-C₁₋₆-Alkyl und -NH-C₁₋₆-Alkyl;
R² ausgewählt ist aus H, -CN, -NO₂ , C₁₋₁₀-Alkyl, C₂₋₁₀-Alkenyl, C₂₋₁₀-Alkinyl, C₀₋₁₀-Alkylen-C₃₋₁₀-Cycloalkyl, C₀₋₁₀-Alkylen-C₃₋₁₀-Heterocycloalkyl, C₀₋₁₀-Alkylen-(5- bis 10-gliedrigem Heteroaryl), C₀₋₁₀-Alkylen-(6- bis 10-gliedrigem Aryl), C₀₋₁₀-Alkylen-(6- bis 10-gliedrigem Heteroaryl), C₀₋₁₀-Alkylen-OR¹¹, C₀₋₁₀-Alkylen-CO₂R¹¹, C₀₋₁₀-Alkylen-C(=O)NR¹¹R¹², C₀₋₁₀-Alkylen-C(=S)NR¹¹R¹², C₀₋₁₀-Alkylen-C(=O)NR¹¹SO₂R¹³, C₀₋₁₀-Alkylen-C(=S)NR¹¹SO₂R¹¹, C₀₋₁₀ -Alkylen-C(=O)R¹¹, C₀₋₁₀-Alkylen-C(=S)R¹¹, C₀₋₁₀-Alkylen-SR¹¹, C₀₋₁₀-Alkylen-SOₓR¹³, C₀₋₁₀-Alkylen-SO₃R¹¹, C₀₋₁₀-Alkylen-SO₂NR¹¹R¹², C₀₋₁₀-Alkylen-NR¹¹C(=O)R¹¹, C₀₋₁₀-Alkylen-NR¹¹ C(=S)R¹¹, C₀₋₁₀-Alkylen-NR¹¹SO₂R¹³, C₀₋₁₀-Alkylen-NR¹¹C(=O)NR¹¹R¹², C₀₋₁₀-Alkylen-NR¹¹ C(=S)NR¹¹R¹², C₀₋₁₀-Alkylen-NR¹¹SO₂NR¹¹R¹², C₀₋₁₀-Alkylen-NR¹¹R¹², wobei Alkyl, Alkenyl, Alkynyl, Alkylen, Cycloalkyl, Heterocycloalkyl, Aryl und Heteroaryl unsubstituiert oder mit 1 bis 7 Substituenten substituiert ist, die unabhängig voneinander aus der Gruppe ausgewählt sind bestehend aus Oxo, CN, -NO₂ , OR¹¹ , O-C₂₋₆-Alkylen-OR¹¹, C₁₋₆-Alkyl, Halogen-C₁₋₆-Alkyl, Halogen, CO₂R¹¹, C(=O)NR¹¹R¹², C(=O)NR¹¹SO₂R¹¹, C(=O)R¹¹, SR¹¹, SOₓR¹¹, SO₃R¹¹, P(=O)(OR¹¹)₂ , SO₂NR¹¹R¹², NR¹¹C(=O)R¹¹, NR¹¹SO₂R¹³, NR¹¹C(=O)NR¹¹R¹², NR¹¹SO₂NR¹¹R¹², C₃₋₁₀-Cycloalkyl, O-C₃₋₁₀-Cycloalkyl, C₃₋₁₀-Heterocycloalkyl, O-C₃₋₁₀-Heterocycloalkyl und NR¹¹R¹² ;
R³ ausgewählt ist aus H, C₁₋₆-Alkyl, Halo-C₁₋₆-Alkyl, -O-C₁₋₆-Alkyl, -O-Halo-C₁₋₆-Alkyl, C₃₋₆-Cycloalkyl und C₃₋₆-Heterocycloalkyl, wobei Alkyl, Cycloalkyl und Heterocycloalkyl gegebenenfalls mit 1 bis 5 Substituenten substituiert sind, die unabhängig voneinander aus Halogen, -CN, OH, Oxo, C₁₋₃-Alkyl, Halogen-C₁₋₃-Alkyl, O-C₁₋₃-Alkyl, O-Halogen-C₁₋₃-Alkyl, SO₂-C₁₋₃-Alkyl und CO₂H ausgewählt sind;
oder R² und R³ zusammen mit dem Stickstoff, an den sie gebunden sind, einen 3- bis 8-gliedrigen Ring vervollständigen, der Kohlenstoffatome und gegebenenfalls 1 oder 2 Heteroatome, ausgewählt aus O, S oder N, enthält, wobei der Ring unsubstituiert oder mit 1 bis 4 Substituenten substituiert ist, die unabhängig voneinander ausgewählt sind aus der Gruppe bestehend aus Halogen, -CN, -NO₂, OH, Oxo, C₁₋₃-Alkyl, Halogen-C₁₋₃-Alkyl, O-C₁₋₃-Alkyl, O-Halogen-C₁₋₃-Alkyl, SO₂-C₁₋₃-Alkyl und CO₂H;
R⁴ ausgewählt ist aus H, C₁₋₆-Alkyl, C₁₋₆-Acyl, C₂₋₆-Alkenyl, C₃₋₈-Cycloalkyl und C₃₋₈-Heterocycloalkyl, wobei Alkyl, Acyl, Alkenyl, Cycloalkyl und Heterocycloalkyl gegebenenfalls mit 1 bis 5 Substituenten substituiert sind, die unabhängig voneinander ausgewählt sind aus Halogen, -CN, OH, Oxo, C₁₋₃-Alkyl, Halogen-C₁₋₃-Alkyl, O-C₁₋₃-Alkyl und O-Halogen-C₁₋₃-Alkyl;
R⁵ und R⁶ und R⁵' und R⁶' unabhängig voneinander ausgewählt sind aus H, Halogen, C₁₋₆-Alkyl, NH₂, NHC₁₋₆-Alkyl, N(C₁₋₆-Alkyl)₂ und C₀₋₆-Alkylen-C(=O)NH₂;
oder R⁵ und R⁶ und R⁵' und R⁶' unabhängig voneinander zusammen mit dem Kohlenstoff, an den sie gebunden sind, einen 3- bis 8-gliedrigen Ring vervollständigen, der Kohlenstoffatome und gegebenenfalls 1 oder 2 Heteroatome, ausgewählt aus O, S oder N, enthält, wobei der Ring unsubstituiert oder mit 1 bis 4 Substituenten substituiert ist, die unabhängig voneinander ausgewählt sind aus der Gruppe bestehend aus Halogen, -CN, -NO₂ , OH, Oxo, C₁₋₃-Alkyl, Halogen-C₁₋₃-Alkyl, O-C₁₋₃-Alkyl, O-Halogen-C₁₋₃-Alkyl, SO₂-C₁₋₃-Alkyl und CO₂H;
oder R⁵ und R⁵ ' und R⁶ und R⁶ ' unabhängig voneinander zusammen mit den beiden benachbarten Kohlenstoffatomen, an die sie gebunden sind, einen 3- bis 8-gliedrigen Ring vervollständigen, der Kohlenstoffatome und gegebenenfalls 1 oder 2 Heteroatome, ausgewählt aus O, S oder N, enthält, wobei der Ring unsubstituiert oder mit 1 bis 4 Substituenten substituiert ist, die unabhängig voneinander ausgewählt sind aus der Gruppe bestehend aus Halogen, -CN, -NO₂, OH, Oxo, C₁₋₃-Alkyl, Halogen-C₁₋₃-Alkyl, O-C₁₋₃-Alkyl, O-Halogen-C₁₋₃-Alkyl, SO₂-C₁₋₃-Alkyl und CO₂H;
oder einer der Reste R⁵, R⁵', R⁶ und R⁶' zusammen mit R⁷ einen 8- bis 11-gliedrigen bicyclischen Ring bilden kann, der gegebenenfalls mit 1 bis 4 Substituenten substituiert ist, die unabhängig voneinander ausgewählt sind aus der Gruppe bestehend aus Halogen, -CN, -NO₂, OH, Oxo, C₁₋₃-Alkyl, Halogen-C₁₋₃-Alkyl, O-C₁₋₃-Alkyl, O-Halogen-C₁₋₃-Alkyl, SO₂-C₁₋₃-Alkyl und CO₂H;
R⁷ ausgewählt ist aus einem 6-gliedrigen Aryl und 5- oder 6-gliedrigen Heteroaryl, wobei Aryl und Heteroaryl gegebenenfalls mit 1 bis 4 Substituenten substituiert sind, die unabhängig voneinander ausgewählt sind aus Halogen, -CN, -NO₂, OH, C₁₋₆-Alkyl, O-C₁₋₆-Alkyl, C₃₋₆-Cycloalkyl, O-C₃₋₆-Cycloalkyl, C₃₋₆-Heterocycloalkyl, O-C₃₋₆-Heterocycloalkyl, SO_{y}-C₁₋₆-Alkyl, CO₂H, C(=O)O-C₁₋₆-Alkyl, 6- bis 10-gliedrigem Aryl, 5- bis 10-gliedrigem Heteroaryl, O-(6- bis 10-gliedrigem Aryl) und O-(5- bis 10-gliedrigem Heteroaryl), wobei Alkyl, Cycloalkyl, Heterocycloalkyl, Aryl und Heteroaryl gegebenenfalls mit 1 bis 5 Substituenten substituiert sind, die unabhängig voneinander ausgewählt sind aus Halogen, -CN, -NO₂, OH, R¹³, OR¹³, CO₂R¹¹, NR¹¹R¹², C(=O)R¹¹, C(=S)R¹¹, C(=O)NR¹¹R¹², NR¹¹C(=O)NR¹¹R¹², NR¹¹C(=O)OR¹³, OC(=O)NR¹¹R¹², C(=S)NR¹¹R¹², NR¹¹C(=S)NR¹¹R¹², NR¹¹C(=S)OR¹³, OC(=S)NR¹¹R¹², SO_{y}-C₁₋₆-Alkyl, SO_{y}-Halogen-C₁₋₆-Alkyl, SR¹¹, SOₓR¹³, SO₃R¹¹, SO₂NR¹¹R¹², NR¹¹SO₂R¹³ und NR¹¹SO₂NR¹¹R¹²;
R⁸ ausgewählt ist aus H, -CN, -NO₂, C₁₋₁₀-Alkyl, C₂₋₁₀-Alkenyl, C₂₋₁₀-Alkinyl, C₀₋₁₀-Alkylen-C₃₋₁₀-Cycloalkyl, C₀₋₁₀-Alkylen-C₃₋₁₀-Heterocycloalkyl, C₀₋₁₀-Alkylen-(5- bis 10-gliedrigem Heteroaryl), C₀₋₁₀-Alkylen-(6- bis 10-gliedrigem Aryl), C₀₋₁₀-Alkylen-(6- bis 10-gliedrigem Heteroaryl), C₀₋₁₀-Alkylen-OR¹¹, C₀₋₁₀-Alkylen-CO₂R¹¹, C₀₋₁₀-Alkylen-C(=O)NR¹¹R¹², C₀₋₁₀-Alkylen-C(=S)NR¹¹R¹², C₀₋₁₀-Alkylen-C(=O)NR¹¹SO₂R¹³, C₀₋₁₀-Alkylen-C(=S)NR¹¹SO₂R¹¹, C₀₋₁₀ -Alkylen-C(=O)R¹¹, C₀₋₁₀-Alkylen-C(=S)R¹¹, C₀₋₁₀-Alkylen-SR¹¹, C₀₋₁₀-Alkylen-SOₓR¹³, C₀₋₁₀-Alkylen-SO₃R¹¹, C₀₋₁₀-Alkylen-SO₂NR¹¹R¹², C₀₋₁₀-Alkylen-NR¹¹C(=O)R¹¹, C₀₋₁₀-Alkylen-NR¹¹C(=S)R¹¹, C₀₋₁₀-Alkylen-NR¹¹SO₂R¹¹, C₀₋₁₀-Alkylen-NR¹¹C(=O)NR¹¹R¹², C₀₋₁₀-Alkylen-NR¹¹C(=S)NR¹¹R¹², C₀₋₁₀-Alkylen-NR¹¹-SO₂-NR¹¹R¹², C₀₋₁₀-Alkylen-NR¹¹R¹², wobei Alkyl, Alkenyl, Alkynyl, Alkylen, Cycloalkyl, Heterocycloalkyl, Aryl und Heteroaryl unsubstituiert oder mit 1 bis 7 Substituenten substituiert sind, die unabhängig voneinander ausgewählt sind aus der Gruppe Oxo, CN, -NO₂, OR¹¹, O-C₂₋₆-Alkylen-OR¹¹, C₁₋₆-Alkyl, Halogen-C₁₋₆-Alkyl, Halogen, CO₂R¹¹, CONR¹¹R¹², CONR¹¹SO₂R¹¹, COR¹¹, SOₓR¹¹, SO₃H, PO(OH)₂, SO₂NR¹¹R¹², NR¹¹COR¹¹, NR¹¹SO₂R¹¹, NR¹¹-CO-NR¹¹R¹², NR¹¹-SO₂-NR¹¹R¹², C₃₋₁₀-Cycloalkyl, O-C₃₋₁₀-Cycloalkyl, C₃₋₁₀-Heterocycloalkyl, O-C₃₋₁₀-Heterocycloalkyl und NR¹¹R¹² ;
R⁹ ausgewählt ist aus C₁₋₁₀-Alkyl, C₂₋₁₀-Alkenyl, C₂₋₁₀-Alkinyl, C₀₋₁₀-Alkylen-C₃₋₁₀-Cycloalkyl, C₀₋₁₀-Alkylen-C₃₋₁₀-Heterocycloalkyl, C₀₋₁₀-Alkylen-(5- bis 10-gliedrigem Heteroaryl), C₀₋₁₀-Alkylen-(6- bis 10-gliedrigem Aryl), C₀₋₁₀-Alkylen-(6- bis 10-gliedrigem Heteroaryl), C₀₋₁₀-Alkylen-OR¹¹, C₀₋₁₀-Alkylen-CO₂R¹¹, C₀₋₁₀-Alkylen-C(=O)NR¹¹R¹², C₀₋₁₀-Alkylen-C(=S)NR¹¹R¹², C₀₋₁₀-Alkylen-C(=O)NR¹¹SO₂R¹³, C₀₋₁₀-Alkylen-C(=S)NR¹¹SO₂R¹¹, C₀₋₁₀-Alkylen-C(=O)R¹¹, C₀₋₁₀-Alkylen-C(=S)R¹¹, C₀₋₁₀-Alkylen-SR¹¹, C₀₋₁₀-Alkylen-SOₓR¹³, C₀₋₁₀-Alkylen-SO₃R¹¹, C₀₋₁₀-Alkylen-SO₂ NR¹¹R¹², C₀₋₁₀-Alkylen-NR¹¹C(=O)R¹¹, C₀₋₁₀-Alkylen-NR¹¹C(=S)R¹¹, C₀₋₁₀-Alkylen-NR¹¹SO₂R¹³, C₀₋₁₀-Alkylen-NR¹¹C(=O)NR¹¹R¹², C₀₋₁₀-Alkylen-NR¹¹C(=S)NR¹¹R¹², C₀₋₁₀-Alkylen-NR¹¹SO₂NR¹¹R¹², C₀₋₁₀-Alkylen-NR¹¹R¹², wobei Alkyl, Alkenyl, Alkinyl, Alkylen, Cycloalkyl, Heterocycloalkyl, Aryl und Heteroaryl unsubstituiert oder mit 1 bis 7 Substituenten substituiert sind, die unabhängig voneinander ausgewählt sind aus der Gruppe bestehend aus Oxo, CN, -NO₂, OR¹¹, O-C₂₋₆-Alkylen-OR¹¹, C₁₋₆-Alkyl, Halogen-C₁₋₆-Alkyl, Halogen, CO₂R¹¹, C(=O)NR¹¹ R¹², C(=O)NR¹¹SO₂R¹¹, C(=O)R¹¹, SR¹¹, SOₓR¹¹, SO₃R¹¹, P(=O)(OR¹¹)₂, SO₂NR¹¹R¹², NR¹¹C(=O)R¹¹, NR¹¹SO₂R¹³, NR¹¹C(=O)NR¹¹R¹², NR¹¹SO₂NR¹¹R¹², C₃₋₁₀-Cycloalkyl, O-C₃₋₁₀-Cycloalkyl, C₃₋₁₀-Heterocycloalkyl, O-C₃₋₁₀-Heterocycloalkyl und NR¹¹R¹² ;
R¹¹ unabhängig ausgewählt ist aus H, C₁₋₆-Alkyl, C₀₋₆-Alkylen-C₃₋₁₀-Cycloalkyl und C₀₋₆-Alkylen-C₃₋₁₀-Heterocycloalkyl, wobei Alkyl, Alkylen, Cycloalkyl und Heterocycloalkyl unsubstituiert oder mit 1 bis 6 Substituenten substituiert sind, die unabhängig voneinander ausgewählt sind aus der Gruppe bestehend aus Halogen, -CN, OH, Oxo, C₁₋₃-Alkyl, Halogen-C₁₋₃-Alkyl, O-C₁₋₃ -Alkyl, O-Halogen-C₁₋₃-Alkyl, NH₂, NH(C₁₋₃-Alkyl), N(C₁₋₃-Alkyl)₂, C₃₋₆-Heterocycloalkyl, C₃₋₆-Cycloalkyl, SO₂-NHC₁₋₃-Alkyl, SO₂-N(C₁₋₃-Alkyl)₂ und SO₂-C₁₋₃-Alkyl, wobei Cycloalkyl und Heterocycloalkyl unsubstituiert oder mit 1 bis 3 Substituenten substituiert sind, die unabhängig voneinander aus der Gruppe ausgewählt sind bestehend aus F, OH, Oxo, CH₃, CHF₂ und CF₃;
R¹² unabhängig ausgewählt ist aus H, C₁₋₆-Alkyl, Halogen-C₁₋₆-Alkyl und C₃₋₆-Cycloalkyl;
oder R¹¹ und R¹² zusammen mit dem Stickstoff, an den sie gebunden sind, einen 3- bis 8-gliedrigen Ring vervollständigen, der Kohlenstoffatome und gegebenenfalls 1 oder 2 Heteroatome, ausgewählt aus O, S oder N, enthält, wobei der Ring unsubstituiert oder mit 1 bis 4 Substituenten substituiert ist, die unabhängig voneinander ausgewählt sind aus der Gruppe bestehend aus Halogen, -CN, -NO₂, OH, Oxo, C₁₋₃-Alkyl, Halogen-C₁₋₃-Alkyl, O-C₁₋₃-Alkyl, O-Halogen-C₁₋₃-Alkyl, SO₂-C₁₋₃-Alkyl und CO₂H;
R¹³ unabhängig ausgewählt ist aus C₁₋₆-Alkyl, C₀₋₆-Alkylen-C₃₋₁₀-Cycloalkyl und C₀₋₆-Alkylen-C₃₋₁₀ -Heterocycloalkyl, wobei Alkyl, Alkylen, Cycloalkyl und Heterocycloalkyl unsubstituiert oder mit 1 bis 6 Substituenten substituiert sind, die unabhängig ausgewählt sind aus der Gruppe bestehend aus Halogen, -CN, OH, Oxo, C₁₋₃-Alkyl, Halogen-C₁₋₃-Alkyl, O-C₁₋₃-Alkyl, O-Halogen-C₁₋₃-Alkyl, NH₂, NH(C₁₋₃-Alkyl), N(C₁₋₃-Alkyl)₂, C₃₋₆-Heterocycloalkyl, C₃₋₆-Cycloalkyl, SO₂-NHC₁₋₃-Alkyl, SO₂-N(C₁₋₃-Alkyl)₂ und SO₂-C₁₋₃-Alkyl, wobei Cycloalkyl und Heterocycloalkyl unsubstituiert oder mit 1 bis 3 Substituenten substituiert ist, die unabhängig voneinander ausgewählt sind aus der Gruppe bestehend aus F, OH, Oxo, CH₃, CHF₂ und CF₃;
n wird aus 0 und 1 ausgewählt;
x unabhängig ausgewählt ist aus 1 und 2;
y unabhängig ausgewählt ist aus 0, 1 und 2;
und wobei gegebenenfalls R¹ mit einem Rest, ausgewählt aus R², R³, R⁸, R⁹ oder R¹¹, verbunden ist, um einen 5- bis 8-gliedrigen Heterocyclus zu bilden, der gegebenenfalls mit 1 bis 4 Substituenten substituiert ist, die unabhängig voneinander ausgewählt sind aus der Gruppe bestehend aus Halogen, -CN, -NO₂, OH, Oxo, C₁₋₃-Alkyl, Halogen-C₁₋₃-Alkyl, O-C₁₋₃-Alkyl, O-Halogen-C₁₋₃-Alkyl, SO₂-C₁₋₃-Alkyl und CO₂H;
oder eine Struktur der folgenden Formel aufweist 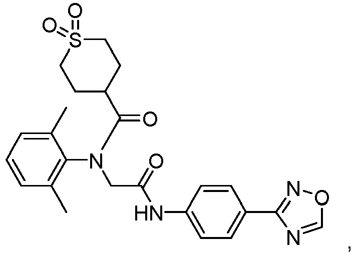
oder ein Solvat, eine Formulierung oder ein pharmazeutisch akzeptables Salz davon.

2. Helicase-Primase-Inhibitoren für die Verwendung nach Anspruch 1, mit einer Struktur der Formel (la) oder (Ib) 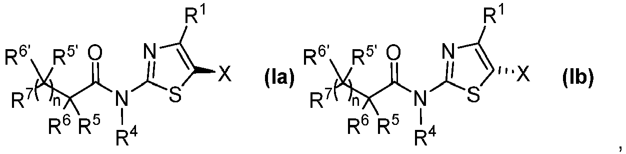 wobei
in der Formel (la) X 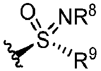 und in der Formel (Ib) X 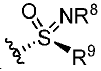 ist, oder ein Solvat, eine Formulierung oder ein pharmazeutisch akzeptables Salz davon.

3. Helicase-Primase-Inhibitoren für die Verwendung nach Anspruch 1 oder 2, die durch die folgende Formel dargestellt werden 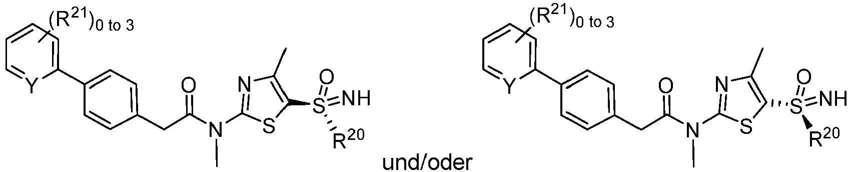 wobei
R²⁰ ausgewählt ist aus C₁₋₄-Alkyl und C₃₋₆-Cycloalkyl, wobei Alkyl und Cycloalkyl unsubstituiert oder mit 1 bis 3 Substituenten substituiert ist, die unabhängig voneinander ausgewählt sind aus der Gruppe bestehend aus F und Me;
R²¹ ausgewählt ist aus F, CI, OH, Me, OMe, CHF₂, CF₃, OCHF₂, OCF₃; und
Y ausgewählt ist aus Stickstoff oder Kohlenstoff,
oder ein Solvat, eine Formulierung oder ein pharmazeutisch akzeptables Salz davon.

4. Helicase-Primase-Inhibitoren zur Verwendung nach einem der Ansprüche 1 bis 3, die ausgewählt sind aus der Gruppe bestehend aus 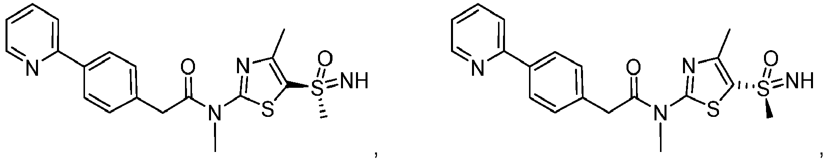 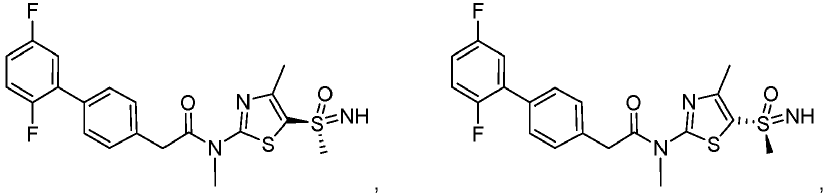 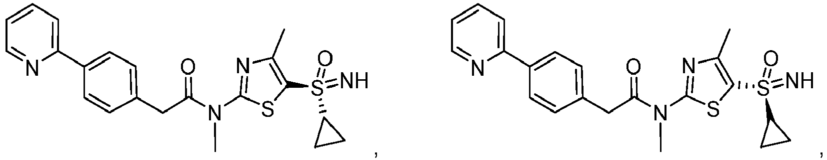 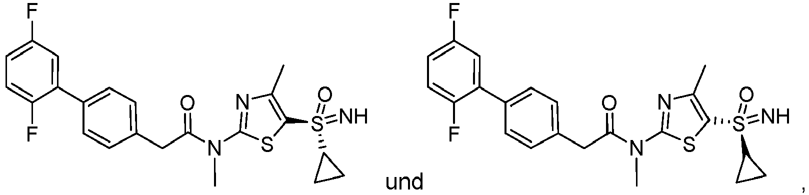 oder ein Solvat, eine Formulierung oder ein pharmazeutisch akzeptables Salz davon.

5. Helicase-Primase-Inhibitoren für die Verwendung nach einem der Ansprüche 1 bis 4, mit einer Struktur ausgewählt aus 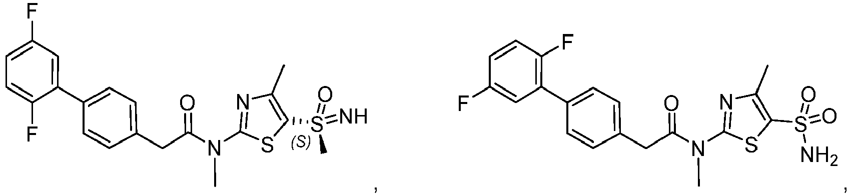 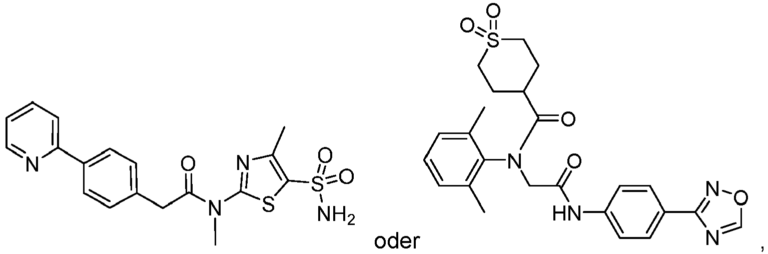 oder ein Solvat, eine Formulierung oder ein pharmazeutisch akzeptables Salz davon.

6. Helicase-Primase-Inhibitor zur Verwendung nach einem der Ansprüche 1 bis 5, welcher folgender ist 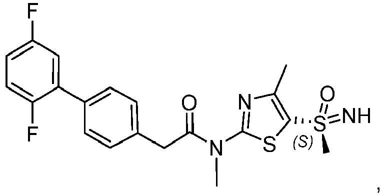 oder ein Solvat, eine Formulierung oder ein pharmazeutisch akzeptables Salz davon.

7. Pharmazeutische Zusammensetzung zur Verwendung als Antidot in einer Kombinationstherapie mit onkolytischen Herpesviren zur Behandlung von Krebs, enthaltend mindestens einen Helikase-Primase-Inhibitor, wie in einem der vorhergehenden Ansprüche definiert, der die Aktivität von onkolytischen Herpesviren, die empfindlich auf die in der Krebstherapie verwendeten Inhibitoren reagieren, kontrolliert, moduliert, hemmt oder abschaltet, und der außerdem mindestens einen pharmazeutisch akzeptablen Träger und/oder Hilfsstoff und/oder mindestens einen weiteren Wirkstoff, wie antiviral aktive oder immunmodulierende Verbindungen, einschließlich Checkpoint-Inhibitoren, enthalten kann, der bei der Behandlung einer Krankheit oder Störung wirksam ist, die mit onkolytischen Virusinfektionen verbunden ist, die bei der Behandlung von Krebs eingesetzt werden.

8. Die Helikase-Primase-Inhibitoren oder die pharmazeutische Zusammensetzung für die Verwendung nach einem der vorhergehenden Ansprüche, wobei der zu behandelnde Krebs ein solider Krebs ist, vorzugsweise ist die Krebserkrankung ausgewählt aus Leberkrebs, Lungenkrebs, Dickdarmkrebs, Bauchspeicheldrüsenkrebs, Nierenkrebs, Hirnkrebs, Melanom und Glioblastom usw..

9. Die Helikase-Primase-Inhibitoren oder die pharmazeutische Zusammensetzung für die Verwendung nach einem der vorhergehenden Ansprüche, wobei die Krebstherapie die Infusion, Injektion, intratumorale Injektion oder topische oder transdermale Applikation der onkolytischen Herpesviren oder der mit onkolytischen Herpesviren infizierten Zellen und/oder der Helikase-Primase-Inhibitoren oder der pharmazeutischen Zusammensetzung die diese enthält, umfasst.

10. Die Helikase-Primase-Inhibitoren oder die pharmazeutische Zusammensetzung für die Verwendung nach einem der vorhergehenden Ansprüche, wobei die onkolytischen Herpesviren oder mit onkolytischen Herpesviren infizierten Zellen aus einem onkolytischen Wildtyp, einem klinischen Isolat oder einem Laborherpesvirusstamm oder einem gentechnisch veränderten oder mehrfach mutierten, gegebenenfalls abgeschwächten oder verstärkten onkolytischen Herpesvirus ausgewählt sind.

11. Kit, umfassend mindestens einen der Helikase-Primase-Inhibitoren oder die pharmazeutische Zusammensetzung, wie in einem der vorhergehenden Ansprüche definiert, und mindestens ein onkolytisches Herpesvirus, ausgewählt aus einem Wildtyp, einem Laborstamm, einem klinischen Isolat und einem gentechnisch veränderten oder multimutierten onkolytischen Herpesvirus.

12. Kit nach Anspruch 11 zur Verwendung bei der Behandlung von Krebs, wie in einem der vorhergehenden Ansprüche definiert.

13. Die Helikase-Primase-Inhibitoren oder die pharmazeutische Zusammensetzung, wie in einem der vorhergehenden Ansprüche definiert, zur Verwendung in einer Kombinationstherapie mit onkolytischen Herpesviren zur Behandlung und Prophylaxe von onkolytischen Herpesinfektionen bei Krebspatienten mit potentiell unterdrücktem Immunsystem, wie AIDS-Patienten, Patienten mit einer genetischen oder vererbten Immunschwäche, Transplantationspatienten; bei neugeborenen Kindern und Säuglingen; bei Herpes-positiven Patienten, insbesondere bei onkolytischen Herpes-simplex-positiven Patienten, zur Unterdrückung des Wiederauftretens oder der onkolytischen Virusausscheidung; bei Patienten, insbesondere bei Herpes-positiven Patienten, insbesondere bei onkolytischen Herpes-simplex-positiven Patienten, die gegen eine nukleosidische antivirale Therapie wie Acyclovir, Penciclovir, Famciclovir, Ganciclovir, Valacyclovir und/oder Foscarnet oder Cidofovir resistent sind.

## Revendications

1. Inhibiteurs de l'hélicase primase pour l'utilisation dans une thérapie combinée avec des herpèsvirus oncolytiques pour le traitement du cancer, dans laquelle les inhibiteurs de l'hélicase primase sont choisis parmi les composés ayant une structure de la formule (I) 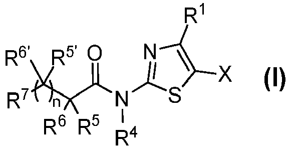 un énantiomère, un diastéréomère, un tautomère, un *N-oxyde,* un solvate, une formulation ou un sel pharmaceutiquement acceptable de ces composés, dans laquelle
X est choisi parmi 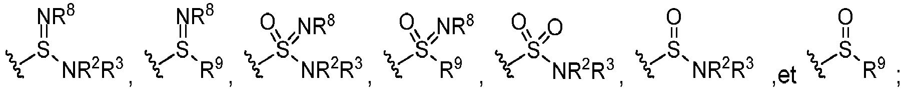
R¹ est choisi parmi H, un halogène, C₁₋₆-alkyle, halo-C₁₋₆-alkyle, hydroxy-C₁₋₆-alkyle, C₃₋₆-cycloalkyle, halo-C₃₋₆-cycloalkyle, -O-C₁₋₆-alkyle, -O-halo-C₁₋₆-alkyle et -NH-C₁₋₆-alkyle;
R² est choisi parmi H, -CN, -NO₂, C₁₋₁₀-alkyle, C₂₋₁₀- alkényle, C₂₋₁₀- alkynyle, C₀₋₁₀-alkylène-C₃₋₁₀-cycloalkyle, C₀₋₁₀-alkylène-C₃₋₁₀-hétérocycloalkyle, C₀₋₁₀-alkylène-(hétéroaryle de 5 à 10 chaînons), C₀₋₁₀-alkylène-(aryle de 6 à 10 chaînons), C₀₋₁₀-alkylène-(hétéroaryle de 6 à 10 chaînons), C₀₋₁₀-alkylène-OR¹¹, C₀₋₁₀-alkylène-CO₂R¹¹, C₀₋₁₀-alkylène-C(=O)NR¹¹R¹², C₀₋₁₀-alkylène-C(=S)NR¹¹R¹², C₀₋₁₀-alkylène-C(=O)NR¹¹SO₂R¹³, C₀₋₁₀-alkyléne-C(=S)NR¹¹SO₂R¹¹, C₀₋₁₀-alkylène-C(=O)R¹¹, C₀₋₁₀-alkylène-C(=S)R¹¹, C₀₋₁₀-alkylène-SR¹¹, C₀₋₁₀-alkylène-SOₓR¹³, C₀₋₁₀-alkylène-SO₃R¹¹, C₀₋₁₀-alkylène-SO₂NR¹¹R¹², C₀₋₁₀-alkylène-NR¹¹C(=O)R¹¹, C₀₋₁₀-alkylène-NR¹¹C(=S)R¹¹, C₀₋₁₀-alkylène-NR¹¹SO₂R¹³, C₀₋₁₀-alkylène-NR¹¹C(=O)NR¹¹R¹², C₀₋₁₀-alkylène-NR¹¹C(=S)NR¹¹R¹², C₀₋₁₀-alkyléne-NR¹¹SO₂NR¹¹R¹², C₀₋₁₀-alkylène-NR¹¹R¹², dans lesquels l'alkyle, l'alkényle, l'alkynyle, l'alkylène, le cycloalkyle, l'hétérocycloalkyle, l'aryle et l'hétéroaryle sont non substitués ou substitués par 1 à 7 substituants choisis indépendamment dans le groupe constitué par oxo, CN, -NO₂, OR¹¹, O-C₂₋₆-alkylène-OR¹¹, C₁₋₆-alkyle, halo-C₁₋₆-alkyle, halogène, CO₂R¹¹, C(=O)NR¹¹R¹², C(=O)NR¹¹SO₂R¹¹, C(=O)R¹¹, SR¹¹, SOₓR¹¹, SO₃R¹¹, P(=O)(OR¹¹)₂, SO₂NR¹¹R¹², NR¹¹C(=O)R¹¹, NR¹¹SO₂R¹³, NR¹¹C(=O)NR¹¹R¹², NR¹¹SO₂NR¹¹R¹², C₃₋₁₀-cycloalkyle, O-C₃₋₁₀-cycloalkyle, C₃₋₁₀-hétérocycloalkyle, O-C₃₋₁₀-hétérocycloalkyle et NR¹¹R¹²;
R³ est choisi parmi H, C₁₋₆-alkyle, halo-C₁₋₆-alkyle, -O-C₁₋₆-alkyle, -O-halo-C₁₋₆-alkyle, C₃₋₆-cycloalkyle et C₃₋₆-hétérocycloalkyle, dans lesquels l'alkyle, le cycloalkyle et l'hétérocycloalkyle sont optionnellement substitués par 1 à 5 substituants choisis indépendamment parmi l'halogène, -CN, OH, oxo, C₁₋₃-alkyle, halo-C₁₋₃-alkyle, O-C₁₋₃-alkyle, O-halo-C₁₋₃-alkyle, SO₂-C₁₋₃-alkyle et CO₂H;
ou R² et R³, lorsqu'ils sont combinés à l'azote auquel ils sont attachés, constituent un anneau de 3 à 8 chaînons contenant des atomes de carbone et optionnellement 1 ou 2 hétéroatomes choisis parmi O, S ou N, l'anneau étant non substitué ou substitué par 1 à 4 substituants choisis indépendamment dans le groupe constitué par l'halogène, -CN, -NO₂, OH, oxo, C₁₋₃-alkyle, halo-C₁₋₃-alkyle, O-C₁₋₃-alkyle, O-halo-C₁₋₃-alkyle, SO₂-C₁₋₃-alkyle et CO₂H;
R⁴ est choisi parmi H, C₁₋₆-alkyle, C₁₋₆-acyle, C₂₋₆-alkényle, C₃₋₈-cycloalkyle et C₃₋₈-hétérocycloalkyle, dans lesquels l'alkyle, l'acyle, l'alkényle, le cycloalkyle et l'hétérocycloalkyle sont optionnellement substitués par 1 à 5 substituants choisis indépendamment parmi l'halogène, -CN, OH, oxo, C₁₋₃-alkyle, halo-C₁₋₃-alkyle, O-C₁₋₃-alkyle et O-halo-C₁₋₃-alkyle;
R⁵ et R⁶ et R^{5'} et R^{6'} sont choisis indépendamment parmi H, halogène, C₁₋₆-alkyle, NH₂, NHC₁₋₆-alkyle, N(C₁₋₆-alkyle)₂ et C₀₋₆-alkylène-C(=O)NH₂;
ou R⁵ et R⁶ et R⁵ ' et R⁶ ' indépendamment, lorsqu'ils sont combines avec le carbone auquel ils sont attachés, constituent un anneau de 3 à 8 chaînons contenant des atomes de carbone et contenant optionnellement 1 ou 2 hétéroatomes choisis parmi O, S ou N, dans lequel l'anneau est non substitué ou substitué par 1 à 4 substituants choisis indépendamment dans le groupe constitué par l'halogène, -CN, -NO₂ , OH, oxo, C₁₋₃-alkyle, halo-C₁₋₃-alkyle, O-C₁₋₃-alkyle, O-halo-C₁₋₃-alkyle, SO₂-C₁₋₃-alkyle et CO₂H;
ou R⁵ et R^{5'} et R⁶ et R^{6'} indépendamment, lorsqu'ils sont combinés avec les deux carbones adjacents auxquels ils sont attachés, constituent un anneau de 3 à 8 chaînons contenant des atomes de carbone et contenant optionnellement 1 ou 2 hétéroatomes choisis parmi O, S ou N, dans lequel l'anneau est non substitué ou substitué par 1 à 4 substituants choisis indépendamment dans le groupe constitué par l'halogène, -CN, -NO₂, OH, oxo, C₁₋₃-alkyle, halo-C₁₋₃-alkyle, O-C₁₋₃-alkyle, O-halo-C₁₋₃-alkyle, SO₂-C₁₋₃-alkyle et CO₂H;
ou tout R⁵, R^{5'}, R⁶ et R^{6'} peut former un anneau bicyclique de 8 à 11 chaînons avec R⁷, qui est optionnellement substitué par 1 à 4 substituants choisis indépendamment dans le groupe constitué d'halogène, -CN, -NO₂, OH, oxo, C₁₋₃-alkyle, halo-C₁₋₃-alkyle, O-C₁₋₃-alkyle, O-halo-C₁₋₃-alkyle, SO₂-C₁₋₃-alkyle et CO₂H;
R⁷ est choisi parmi un aryle à 6 chaînons et un hétéroaryle à 5 ou 6 chaînons, dans lesquels l'aryle et l'hétéroaryle sont optionnellement substitués par 1 à 4 substituants choisis indépendamment parmi l'halogène, -CN, -NO₂, OH, C₁₋₆-alkyle, O-C₁₋₆-alkyle, C₃₋₆-cycloalkyle, O-C₃₋₆-cycloalkyle, C₃₋₆-hétérocycloalkyle, O-C₃₋₆-hétérocycloalkyle, SO_{y-}C₁₋₆-alkyle, CO₂H, C(=O)O-C₁₋₆-alkyle, aryle à 6-10 chaînons, hétéroaryle à 5-10 chaînons, O-(aryle de 6 à 10 chaînons) et O-(hétéroaryle de 5 à 10 chaînons), dans lesquels l'alkyle, le cycloalkyle, l'hétérocycloalkyle, l'aryle et l'hétéroaryle sont optionnellement substitués par 1 à 5 substituants choisis indépendamment parmi l'halogène, -CN, -NO₂, OH, R¹³, OR¹³, CO₂R¹¹, NR¹¹R¹², C(=O)R¹¹, C(=S)R¹¹, C(=O)NR¹¹R¹², NR¹¹C(=O)NR¹¹R¹², NR¹¹C(=O)OR¹³, OC(=O)NR¹¹R¹², C(=S)NR¹¹R¹², NR¹¹C(=S)NR¹¹R¹², NR¹¹C(=S)OR¹³, OC(=S)NR¹¹R¹²; SO_{y}-C₁₋₆-alkyle, SO_{y}-halo-C₁₋₆-alkyle, SR¹¹, SOₓR¹³, SO₃R¹¹, SO₂NR¹¹R¹², NR¹¹SO₂R¹³ et NR₁₁SO₂NR¹¹R¹²;
R⁸ est choisi parmi H, -CN, -NO₂,C₁₋₁₀-alkyle, C₂₋₁₀-alkényle, C₂₋₁₀-alkynyle, C₀₋₁₀-alkylène-C₃₋₁₀-cycloalkyle, C₀₋₁₀-alkylène-C₃₋₁₀-hétérocycloalkyle, C₀₋₁₀-alkylène-(hétéroaryle de 5 à 10 chaînons), C₀₋₁₀-alkylène-(aryle de 6 à 10 chaînons), C₀₋₁₀-alkylène-(hétéroaryle de 6 à 10 chaînons), C₀₋₁₀-alkylène-OR¹¹, C₀₋₁₀-alkylène-CO₂R¹¹, C₀₋₁₀-alkylène-C(=O)NR¹¹R¹², C₀₋₁₀-alkylène-C(=S)NR¹¹R¹², C₀₋₁₀-alkylène-C(=O)NR¹¹SO₂R¹³, C₀₋₁₀-alkyléne-C(=S)NR¹¹SO₂R¹¹, C₀₋₁₀-alkylène-C(=O)R¹¹, C₀₋₁₀-alkylène-C(=S)R¹¹, C₀₋₁₀-alkylène-SR¹¹, C₀₋₁₀-alkylène-SOₓ-R¹³, C₀₋₁₀-alkylène-SO₃R¹¹, C₀₋₁₀-alkylène-SO₂NR¹¹R¹², C₀₋₁₀-alkylène-NR¹¹C(=O)R¹¹, C₀₋₁₀-alkylène-NR¹¹C(=S)R¹¹, C₀₋₁₀-alkylène-NR¹¹SO₂R¹¹, C₀₋₁₀-alkylène-NR¹¹C(=O)NR¹¹R¹², C₀₋₁₀-alkylène-NR¹¹C(=S)NR¹¹R¹², C₀₋₁₀-alkylène-NR¹¹-SO₂-NR¹¹R¹², C₀₋₁₀-alkylène-NR¹¹R¹², dans lesquels alkyle, alkényle, alkynyle, alkylène, cycloalkyle, hétérocycloalkyle, aryle et hétéroaryle sont non substitués ou substitués par 1 à 7 substituants choisis indépendamment dans le groupe constitué par oxo, CN, -NO₂, OR¹¹, O-C₂₋₆-alkylène-OR¹¹,C₁₋₆-alkyle, halo-C₁₋₆-alkyle, halogène, CO₂R¹¹, CONR¹¹R¹², CONR¹¹SO₂R¹¹, COR¹¹, SOₓR¹¹, SO₃H, PO(OH)₂, SO₂NR¹¹R¹², NR¹¹COR¹¹, NR¹¹SO₂R¹¹, NR¹¹-CO-NR¹¹R¹², NR¹¹-SO₂-NR¹¹R¹²,C₃₋₁₀-cycloalkyle, O-C₃₋₁₀-cycloalkyle, C₃₋₁₀-hétérocycloalkyle, O-C₃₋₁₀-hétérocycloalkyle et NR¹¹R¹²;
R⁹ est choisi parmi C₁₋₁₀-alkyle, C₂₋₁₀-alkényle, C₂₋₁₀-alkynyle, C₀₋₁₀-alkylène-C₃₋₁₀-cycloalkyle, C₀₋₁₀-alkylène-C₃₋₁₀-hétérocycloalkyle, C₀₋₁₀-alkylène-(hétéroaryle de 5- à 10-chaînons), C₀₋₁₀-alkylène-(aryle de 6 à 10 chaînons), C₀₋₁₀-alkylène-(hétéroaryle de 6 à 10 chaînons), C₀₋₁₀-alkylène-OR¹¹, C₀₋₁₀-alkylène-CO₂R¹¹, C₀₋₁₀-alkylène-C(=O)NR¹¹R¹², C₀₋₁₀-alkylène-C(=S)NR¹¹R¹², C⁰⁻¹⁰-alkylène-C(=O)NR¹¹SO₂R¹³, C₀₋₁₀-alkylène-C(=S)NR¹¹SO₂R¹¹, C₀₋₁₀-alkylène-C(=O)R¹¹, C₀₋₁₀-alkylène-C(=S)R¹¹,C₀₋₁₀-alkylène-SR¹¹, C₀₋₁₀-alkylène-SOₓR¹³, C₀₋₁₀-alkylène-SO₃R¹¹, C₀₋₁₀-alkylène-SO₂NR¹¹R¹², C₀₋₁₀-alkylène-NR¹¹C(=O)R¹¹, C₀₋₁₀-alkylène-NR¹¹C(=S)R¹¹, C₀₋₁₀-alkylène-NR¹¹SO₂R¹³, C₀₋₁₀-alkylène-NR¹¹C(=O)NR¹¹R¹², C₀₋₁₀-alkylène-NR¹¹C(=S)NR¹¹R¹², C₀₋₁₀-alkylène-NR¹¹SO₂NR¹¹R¹², C₀₋₁₀-alkylène-NR¹¹R¹², dans lesquels l'alkyle, l'alkényle, l'alkynyle, l'alkylène, le cycloalkyle, l'hétérocycloalkyle, l'aryle et l'hétéroaryle sont non substitués ou substitués par 1 à 7 substituants choisis indépendamment dans le groupe constitué par oxo, CN, -NO₂, OR¹¹, O-C₂₋₆-alkylène-OR¹¹, C₁₋₆-alkyle, halo-C₁₋₆-alkyle, halogène, CO₂R¹¹, C(=O)NR¹¹R¹², C(=O)NR¹¹SO₂R¹¹, C(=O)R¹¹, SR¹¹, SOₓR¹¹, SO₃R¹¹, P(=O)(OR¹¹)₂, SO₂NR¹¹R¹², NR¹¹C(=O)R¹¹, NR¹¹SO₂R¹³, NR¹¹C(=O)NR¹¹R¹², NR¹¹SO₂NR¹¹R¹²,C₃₋₁₀-cycloalkyle, O-C₃₋₁₀-cycloalkyle, C₃₋₁₀-hétérocycloalkyle, O-C₃₋₁₀-hétérocycloalkyle et NR¹¹R¹²;
R¹¹ est indépendamment choisi parmi H, C₁₋₆-alkyle, C₀₋₆-alkylène-C₃₋₁₀-cycloalkyle et C₀₋₆-alkylène-C₃₋₁₀-hétérocycloalkyle, dans lesquels l'alkyle, l'alkylène, le cycloalkyle et l'hétérocycloalkyle sont non substitués ou substitués par 1 à 6 substituants indépendamment choisis dans le groupe constitué de l'halogène, CN, OH, oxo,C₁₋₃-alkyle, halo-C₁₋₃-alkyle, OC₁₋₃-alkyle, O-halo-C₁₋₃-alkyle, NH₂, NH(C₁₋₃-alkyle), N(C₁₋₃-alkyle), N(C₃₋₆-hétérocycloalkyle), NH₂, NH(C₁₋₃-alkyle), N(C₁₋₃-alkyle), O-halo-C₁₋₃-alkyle, O-C₁₋₃-alkyle, O-halo-C₁₋₃-alkyle, NH₂, NH(C₁₋₃-alkyle), N(C₁₋₃-alkyle)₂, C₃₋₆-hétérocycloalkyle, C₃₋₆-cycloalkyle, SO₂-NHC₁₋₃-alkyle, SO₂-N(C₁₋₃-alkyle)₂ et SO₂-C₁₋₃-alkyle, dans lesquels le cycloalkyle et l'hétérocycloalkyle sont non substitués ou substitués par 1 à 3 substituants choisis indépendamment dans le groupe constitué par F, OH, oxo, CH₃, CHF₂ et CF₃;
R¹² est indépendamment choisi parmi H, C₁₋₆-alkyle, halo-C₁₋₆-alkyle et C₃₋₆-cycloalkyle;
ou R¹¹ et R¹², lorsqu'ils sont combinés à l'azote auquel ils sont attachés, constituent un anneau de 3 à 8 chaînons contenant des atomes de carbone et optionnellement 1 ou 2 hétéroatomes choisis parmi O, S ou N, dans lequel l'anneau est non substitué ou substitué par 1 à 4 substituants choisis indépendamment dans le groupe consistant en halogène, -CN, -NO₂, OH, oxo, C₁₋₃-alkyle, halo-C₁₋₃-alkyle, O-C₁₋₃-alkyle, O-halo-C₁₋₃-alkyle, SO₂-C₁₋₃-alkyle et CO₂H;
R¹³ est indépendamment choisi parmi C₁₋₆-alkyle, C₀₋₆-alkylène-C₃₋₁₀-cycloalkyle et C₀₋₆-alkylène-C₃₋₁₀-hétérocycloalkyle, dans lesquels l'alkyle, l'alkylène, le cycloalkyle et l'hétérocycloalkyle sont non substitués ou substitués par 1 à 6 substituants choisis indépendamment dans le groupe constitué par halogène, CN, OH, oxo,C₁₋₃-alkyle, halo-C₁₋₃-alkyle, O-C₁₋₃-alkyle, O-halo-C₁₋₃-alkyle, NH₂, NH(C₁₋₃-alkyle), N(C₁₋₃-alkyle)₂, C₃₋₆-hétérocycloalkyle, C₃₋₆-cycloalkyle, SO₂-NHC₁₋₃-alkyle, SO₂-N(C₁₋₃-alkyle)₂ et SO₂-C₁₋₃-alkyle, dans lesquels le cycloalkyle et l'hétérocycloalkyle sont non substitués ou substitués par 1 à 3 substituants choisis indépendamment dans le groupe constitué par F, OH, oxo, CH₃, CHF₂ et CF₃;
n est choisi parmi 0 et 1 ;
x est indépendamment choisi parmi 1 et 2 ;
y est indépendamment choisi parmi 0, 1 et 2 ;
et dans laquelle R¹ est optionnellement lié à un résidu choisi parmi R², R³, R⁸, R⁹ ou R¹¹ pour former un hétérocycle de 5 à 8 chaînons, qui est optionnellement substitué par 1 à 4 substituants choisis indépendamment dans le groupe consistant en halogène, -CN, -NO₂, OH, oxo, C₁₋₃-alkyle, halo-C₁₋₃-alkyle, O-C₁₋₃-alkyle, O-halo-C₁₋₃-alkyle, SO₂-C₁₋₃-alkyle et CO₂H;
ou ayant une structure de la formule suivante 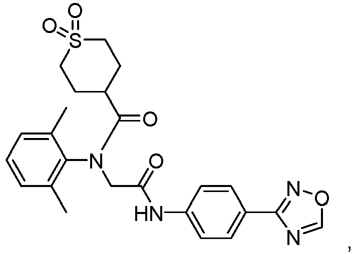
ou un solvate, une formulation ou un sel pharmaceutiquement acceptable de celui-ci.

2. Inhibiteurs de l'hélicase primase pour l'utilisation selon la revendication 1, ayant une structure de la formule **(Ia)** ou **(Ib)** 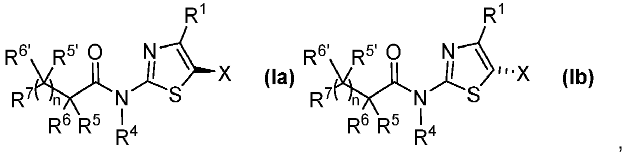 dans lesquelles
dans la formule **(la),** X est 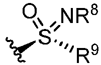 et dans la formule **(Ib),** X est 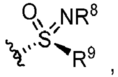 ou un solvate, une formulation ou un sel pharmaceutiquement acceptable de ceux-ci.

3. Inhibiteurs de l'hélicase primase pour l'utilisation selon la revendication 1 ou 2, qui sont représentés par la formule 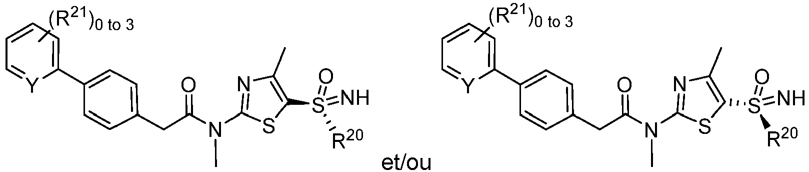 dans lesquelles
R²⁰ est choisi parmi C₁₋₄-alkyle et C₃₋₆-cycloalkyle, dans lesquels l'alkyle et le cycloalkyle sont non substitués ou substitués par 1 à 3 substituants choisis indépendamment dans le groupe consistant en F ou Me;
R²¹ est choisi parmi F, CI, OH, Me, OMe, CHF₂, CF₃, OCHF₂, OCF₃; et
Y est choisi parmi l'azote ou le carbone,
ou un solvate, une formulation ou un sel pharmaceutiquement acceptable de ceux-ci.

4. Inhibiteurs de l'hélicase primase pour l'utilisation selon l'une des revendications 1 à 3, qui sont choisis dans le groupe consistant en 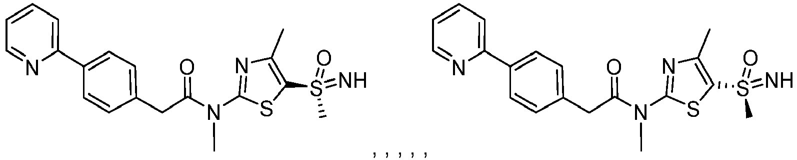 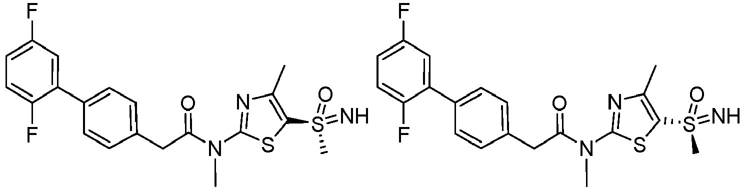 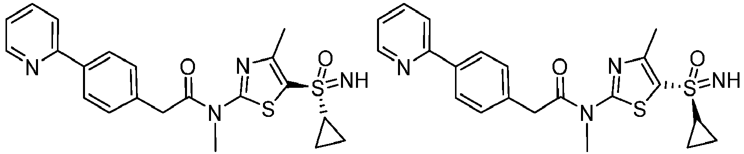 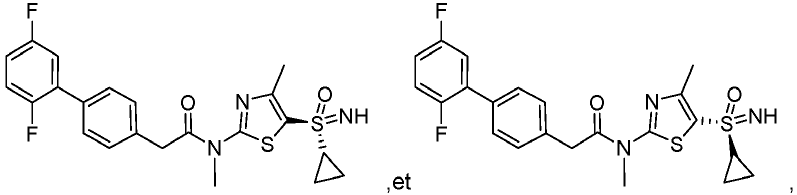 ou un solvate, une formulation ou un sel pharmaceutiquement acceptable de ceux-ci.

5. Inhibiteurs de l'hélicase primase pour l'utilisation selon l'une des revendications 1 à 4, ayant une structure choisie parmi les suivantes 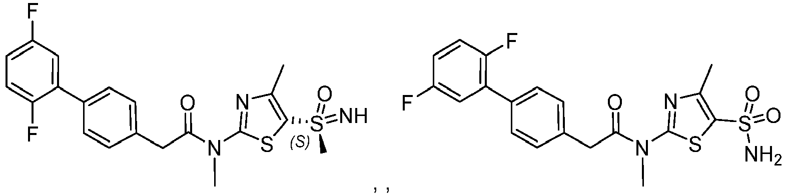 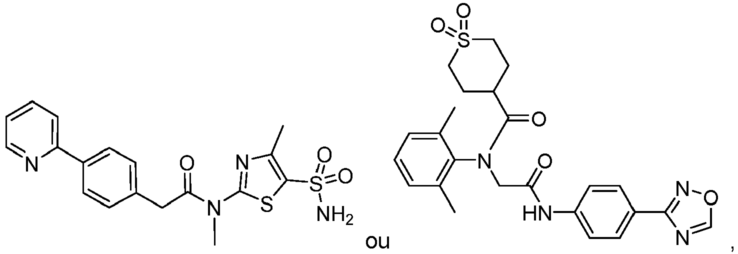 ou un solvate, une formulation ou un sel pharmaceutiquement acceptable de ceux-ci.

6. Inhibiteur de l'hélicase primase pour l'utilisation selon l'une des revendications 1 à 5, qui est 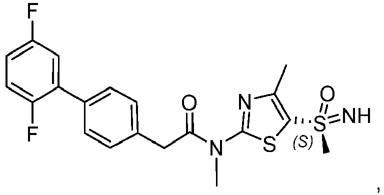 ou un solvate, une formulation ou un sel pharmaceutiquement acceptable de celui-ci.

7. Composition pharmaceutique pour l'utilisation comme antidote dans une thérapie combinée avec des herpèsvirus oncolytiques pour traiter le cancer, qui comprend au moins un inhibiteur d'hélicase primase tel que défini dans l'une quelconque des revendications ci-dessus, qui agit pour contrôler, moduler, inhiber ou arrêter l'activité des herpèsvirus oncolytiques sensibles auxdits inhibiteurs utilisés dans la thérapie du cancer, et qui peut en outre comprendre au moins un support et/ou un excipient pharmaceutiquement acceptable et/ou au moins une autre substance active, comme des composés antiviraux ou immunomodulateurs, y compris des inhibiteurs de points de contrôle, qui est efficace pour traiter une maladie ou un trouble associé à des infections virales oncolytiques utilisées dans le traitement du cancer.

8. Les inhibiteurs de l'hélicase primase ou la composition pharmaceutique pour l'utilisation selon l'une des revendications ci-dessus, dans laquelle le cancer à traiter est un cancer solide, de préférence la maladie cancéreuse est choisie parmi le cancer du foie, le cancer du poumon, le cancer du côlon, le cancer du pancréas, le cancer du rein, le cancer du cerveau, le mélanome et le glioblastome, etc.

9. Les inhibiteurs de l'hélicase primase ou la composition pharmaceutique pour l'utilisation selon l'une des revendications ci-dessus, dans laquelle la thérapie du cancer comprend la perfusion, l'injection, l'injection intratumorale ou l'application topique ou transdermique des herpèsvirus oncolytiques ou des cellules infectées par les herpèsvirus oncolytiques et/ou des inhibiteurs de l'hélicase primase ou de la composition pharmaceutique comprenant ces derniers.

10. Les inhibiteurs de l'hélicase primase ou la composition pharmaceutique pour l'utilisation selon l'une des revendications ci-dessus, dans laquelle les herpèsvirus oncolytiques ou les cellules infectées par les herpèsvirus oncolytiques sont choisis parmi un type sauvage oncolytique, un isolat clinique ou une souche d'herpèsvirus de laboratoire ou un herpèsvirus oncolytique génétiquement modifié ou multi-muté, optionnellement atténué ou boosté.

11. Un kit comprenant au moins un des inhibiteurs de l'hélicase primase ou la composition pharmaceutique définie dans l'une des revendications ci-dessus et au moins un herpèsvirus oncolytique choisi parmi un type sauvage, une souche de laboratoire, un isolat clinique et un herpèsvirus oncolytique génétiquement modifié ou multi-muté.

12. Kit selon la revendication 11 pour l'utilisation dans le traitement du cancer tel que défini dans l'une des revendications ci-dessus.

13. Les inhibiteurs de l'hélicase primase ou la composition pharmaceutique tels que définis dans l'une quelconque des revendications ci-dessus pour l'utilisation dans une thérapie combinée avec des herpèsvirus oncolytiques pour le traitement et la prophylaxie des infections herpétiques oncolytiques chez les patients cancéreux ayant potentiellement un système immunitaire supprimé, comme les patients atteints du SIDA, les patients ayant une immunodéficience génétique ou héréditaire, les patients transplantés; chez les enfants nouveau-nés et les nourrissons; chez les patients atteints d'herpès, en particulier les patients atteints d'herpès-simplexe oncolytique, pour supprimer la récurrence ou l'excrétion virale oncolytique; chez les patients, en particulier les patients atteints d'herpès, en particulier les patients atteints d'herpès-simplexe oncolytique, qui sont résistants à la thérapie antivirale nucléosidique comme l'acyclovir, le penciclovir, le famciclovir, le ganciclovir, le valacyclovir et/ou le foscarnet ou le cidofovir.
